# EUROPEAN PATENT SPECIFICATION

(11) **EP 1 626 709 B1**
(45) Date of publication and mention of the grant of the patent: **05.09.2007**
(21) Application number: 04709870.2
(22) Date of filing: 10.02.2004
(51) Int. Cl.: A61K 31/137, A61K 9/16, A61K 9/20, A61P 25/00

(54) **ENANTIOMERIC AMPHETAMINE COMPOSITIONS FOR THE TREATMENT OF ADHD**
ENANTIOMERE AMPHETAMINE ENTHALTENDE ZUSAMMENSETZUNGEN ZUR BEHANDLUNG VON AUFMERKSAMKEITSDEFIZIT-/HYPERAKTIVITÄTSSTÖRUNG (ADHD)
COMPOSITIONS ENANTIOMERIQUES D'AMPHETAMINE POUR TRAITER L'ADHD

(30) Priority: 10.02.2003 US 445793 P; 29.05.2003 US 473925 P
(43) Date of publication of application: 22.02.2006
(73) Proprietor: Shire BioChem Inc., Laval, Quebec H7V 4A7 (CA)
(72) Inventor: COUCH, Richard, A., Chevy Chase, MD 20815 (US); MICHAELS, A. Shire Pharmaceutical Development Inc., Rockville, MD 20850 (US); HODGKINS, Paul, Exton, PA 19341 (US)
(74) Representative: Harrison Goddard Foote
(86) International application number: PCT/US2004/003821
(87) International publication number: WO 2004/071501

(56) References cited:
- WO-A-00/23055
- WO-A-02/39998
- WO-A-98/40053
- WO-A-03/072105
- WO-A-03/092661
- WO-A-20/04028509
- US-A1- 2002 058 061
- ARNOLD L E ET AL: "Levoamphetamine and dextroamphetamine: comparative efficacy in the hyperkinetic syndrome. Assessment by target symptoms." ARCHIVES OF GENERAL PSYCHIATRY. DEC 1972, vol. 27, no. 6, December 1972 (1972-12), pages 816-822, XP008031904 ISSN: 0003-990X
- ARNOLD L E ET AL: "Levoamphetamine and dextroamphetamine: differential effect on aggression and hyperkinesis in children and dogs." THE AMERICAN JOURNAL OF PSYCHIATRY. FEB 1973, vol. 130, no. 2, February 1973 (1973-02), pages 165-170, XP008031900 ISSN: 0002-953X
- TULLOCH SIMON J ET AL: "SLI381 (Adderall XR), a two-component, extended-release formulation of mixed amphetamine salts: Bioavailability of three test formulations and comparison of fasted, fed, and sprinkled administration." PHARMACOTHERAPY, vol. 22, no. 11, November 2002 (2002-11), pages 1405-1415, XP008031886 ISSN: 0277-0008
- PATRICK KENNERLY S ET AL: "Pharmacology of methylphenidate, amphetamine enantiomers and pemoline in attention-deficit hyperactivity disorder" HUMAN PSYCHOPHARMACOLOGY, vol. 12, no. 6, November 1997 (1997-11), pages 527-546, XP008031884 ISSN: 0885-6222

## Description

This application claims priority to U.S. Provisional Application No. 60/445,793, filed February 10, 2003, and to U.S. Provisional Application No. 60/473,925, filed May 29, 2003.

Amphetamines have long been recognized as stimulators of CNS activity in humans. Amphetamines are believed, for example, to modulate the release and uptake of excitatory neurotransmitters, such as dopamine and norepinephrine. They are used in the treatment of attention deficit hyperactivity disorder (ADHD) in children, adolescents, adults, etc., and may also be used to treat other disorders. They can be administered alone or with other active ingredients. See, generally, WO 00/23055, USSN 60/414401 and USSN 60/431963.

Trade names for amphetamines include e.g., Dexedrene^{®}, DextroStat^{®}, Adderall^{®} immediate-release tablets and Adderall XR^{®} Capsules, (57^{th} Edition of the Physicians Desk Reference, (Thomson PDR)).

In such products, the amphetamine is typically contained in the form of one or more salts, e.g., in Adderall XR^{®} and Adderall^{®} tablets a mixture of dextroamphetamine sulfate, dextroamphetamine saccharate, amphetamine aspartate monohydrate and amphetamine sulfate. As can be seen, the amphetamines have been used in both racemic and enantiomeric form.

ADHD is currently defined as a cognitive/behavioral developmental disorder. Inattentiveness, overactivity, and impulsiveness are presently regarded as the main clinical symptoms. Although current treatments are useful, improvements are still desired, particularly in more beneficially treating any one or more of the symptoms and/or lowering observed side effects, e.g., deterioration of sleep (e.g., increased latency to fall asleep, phase shifts in sleep patterns, typically toward sleeping later), decreased appetite, etc.

This invention relates to a pharmaceutical combination comprising an effective amount for a day of b and d-amphetamine, each in base and/or salt form and each adapted for release such that the molar ratio of 1-amphetamine to d-amphetamine released therefrom in a time period later in the day is higher than said ratio released therefrom in a time period earlier in the day, wherein said time period later in the day is at least one hour following the time period earlier in the day. In various aspects, the invention relates to a pharmaceutical combination, wherein said earlier period is the time before 1:00 pm or 11:00 am of a given day and said later period is the time thereafter (typically, a subsequent release is greater than about 3 hours after a prior release); wherein said amphetamine released in said earlier period comprises substantially only d-amphetamine, racemic amphetamine, or a mixture of d- and 1-amplietamine having more d- than 1-amphetamine; wherein the molar ratio released of d- to 1- amphetamine in said earlier period is 4/1 to 2/1; wherein the molar ratio released of d- to 1- amphetamine in said earlier period is less than 1/1; wherein substantially only d-amphetamine is released in said early period; wherein said amphetamine released in said earlier period comprises a mixture of d- and 1-amphetamine having more 1- than d-amphetamine; wherein said amphetamine released in said later period comprises substantially only l-amphetamine, racemic amphetamine, or a mixture of d- and 1-amphetamine having more 1- than d-amphetamine; wherein said amphetamine released in said later period comprises a mixture of d- and 1-amphetamine having more 1- than d-amphetamine; wherein the molar ratio released of 1- to d-amphetamine in said later period is 2/1 to 6/1; wherein substantially only 1-amphetamine is released in said later period; wherein the total amphetamine dose per day is 1 to 200 mg; which comprises two separate oral dosage forms, one identified to be administered at a time to provide amphetamine release in said earlier period and the other identified to be administered at a time to provide amphetamine release in said later period; which comprises a single oral dosage form which provides amphetamine release in both said earlier and later periods; which comprises a dosage form providing immediate release of d-amphetamine in said earlier period; wherein doses are to be administered individually at different times or are to be administered once in a single staged-release capsule; wherein doses are administered in one or more dosage forms that are either immediate release or pulse release dosage forms and/or sustained or controlled release dosage forms; wherein the sustained or controlled release dosage forms or pills or capsules contain the 1 isomer; wherein two doses of amphetamine are administered to the patient in a day, the first dose having an 1 to d isomer ratio of 1:3 or contains only d isomer, and the later dose having an 1 to d isomer ratio of greater than 1:1 or contains 1 isomer only; or wherein the second dose contains 1 isomer only.

In other aspects, the invention relates to the use of effective amounts of the l-and d-isomers of amphetamine for the manufacture of a medicament for the treatment of ADHD in a patient, wherein the l-and d-isomers of amphetamine are, each independently in free base and/or salt form and each adapted for release such that the molar ratio of l-amphetamine to d-amphetamine released from the medicament in a time period later in the day is higher than said ratio released from the medicament in a time period earlier in the day; and, wherein the molar ratio of the total amount of l-isomer to the total amount of d-isomer administered per day is greater than 1:3. In various aspects, the invention relates to the use of such a composition for treating ADHD, wherein doses are to be administered individually at different times or are to be administered once in a single staged-release capsule; wherein doses are to be administered in one or more dosage forms that are either immediate release or pulse release dosage forms and/or sustained or controlled release dosage forms; wherein the sustained or controlled release dosage forms or pills or capsules contain the l isomer; wherein two doses of amphetamine are to be administered to the patient in a day, the first dose having an l to d isomer ratio of 1:3 or contains only d isomer, and the later dose has an l to d isomer ratio of greater than 1:1 or contains 1 isomer only; or wherein the second dose contains 1 isomer only.

In further aspects, the invention relates to the use of a pharmaceutical combination of claim 1 for the manufacture of a medicament for treating ADHD in a human. In one aspect, this use for treating ADHD is a use wherein inattentiveness later in the day is treated by the l-isomer, preferably as effectively by said l-isomer as with a corresponding molar amount of d-amphetamine, and with lesser side effects, e.g., less sleep deterioration and/or lesser effect on food intake and/or weight loss. Thus, in one embodiment, the enhanced l-ratio later in the day (e.g., in the afternoon) produces a better sleep profile; in another, the enhanced l-ratio later in the day (e.g., at noon), produces lessened effects on eating later, e.g., dinner.

By "early" or "earlier" and "late" and "later," respectively used interchangeably herein, is meant that there are at least two periods in a day (e.g., typically each of a duration of one hour or more, e.g., one to four, five, six, eight hours) wherein the relative amounts released of d- and l-amphetamines are different. There can be three, four or more periods where the released relative amounts of d- and l- amphetamine vary. With respect to any two of these, the chronologically earlier occurring is the early or earlier period and the chronologically subsequent period is the late or later period. In preferred embodiments, the later period of the invention refers to the last such period before bedtime. The ratio of l- to d- amphetamine release in the latest period will be higher than in all the other periods of the day. Typically , an early period is before 2:00 pm, 1:00 pm, 12:00 noon, or 11:00 am, preferably at about 6:00 to 10:00 am, e.g., before or with breakfast, and a late period is thereafter, respectively, e.g., at or after 12 noon, 2:00 pm, 4:00 pm, 6:00 pm, e.g., before or with lunch or dinner, chronologically later occurring late periods typically being more often appropriate for adolescents and adults. It is to be understood that all these terms are relative and defined in relation to a person who has a schedule of sleep that occurs at night. However, alternative schedules, e.g., any number of hours shift in a given person's schedule of sleep or even inverted schedules, e.g., people working night shifts, are also encompassed by the definition, whereby the definitions of the terms "earlier" and "later," etc., shift accordingly to account for the shift in the given person's schedule. For example, the earlier time period would begin with a person's first ingestion of amphetamine dose in his or her "day."

"Release" of amphetamine, e.g., an enantiomer thereof, means the separation of the substance from a dosage form into the surrounding environment, e.g., interior of a human to whom the dosage form has been administered, liquid medium into which the dosage form has been placed for measurement of a dissolution and/or release property, such as rates thereof under selected conditions, often modeling the human interior, such as the stomach, intestine, etc. (e.g., as described in WO 00/23055).

By "substantially only" with respect to a given enantiomer is meant that the embodiment contains less than 2 mole %, preferably less than 1 mole %, and most preferably less than 0.5 mole % or less of the other enantiomer.

"Pharmaceutical combination" refers, for example, to two or more entities (e.g., pharmaceutically acceptable ingredients in a given dosage unit (e.g., tablets, capsules, caplets, patch) ; individual dosage units, e.g., two or more; and/or individual components of a given dosage unit, e.g., layers, beads, compartments, matrices, coatings, shapes) which cooperate on administration to achieve the release properties of this invention. Thus, included are single dosage units, as described elsewhere herein, such as multi-layer, multi-bead, multi-matrix, multi-compartment forms which achieve a controlled (e.g., pulsed, sustained, slow, first order, second order release over an entire day in accordance with this invention; and multiple dosage units (to be simultaneously or more often sequentially administered), typically associated together, e.g., in the form of a kit containing the multiple units, identified in a manner which indicates the time of day or relative time order in which the units are to be administered. One of many such kits is a conventional blister pack (or other pouch or container system) containing the multiple units and instructions or notices or indicia regarding the timing and order of administration. Other manner of identifying the time of day and/or order of administration can include unit shape, color, size, etc and combinations thereof.

"Effective amounts" has its conventional definition, e.g., amounts sufficient to have a beneficial effect on the disease state being treated, e.g., ADHD in children, adolescents or adults. Typical total daily doses are in the range of 1 to 200 mg, more typically 5 to 150 mg, preferably, 10 to 100 mg, e.g., 20 mg, 30 mg, 40 mg, 50 mg, 60 mg. Dosage amounts released in given dosage forms over given time intervals in accordance with this invention will total over a day to a value in these ranges typically. The molar ratios to be achieved in accordance with this invention include all ratios from substantially only either of the enantiomers to any other ratio in between these two extremes, as long as the features of the invention are met.

This invention includes all possible regimens of administering the two enantiomers of amphetamine, e.g., administration times during a day for either or both, relative doses, all combinations of the two enantiomers, etc, as long as the features of the invention are met. Thus, modes include administering more of the d-isomer early in the day (e.g., 6:00 am to 11:00 am), e.g., only d-isomer or a molar ratio of d/l of 1.5 to 5/1, or higher or lower, (e.g., 3/1 as in Adderall^{®}), and then administering a higher ratio of the 1-isomer later in the day (e.g., 12:00 noon or later, e.g., 4:00 pm, 6:00 pm, etc.), e.g., only 1-isomer, or a molar ratio of l/d of 1.5 to 5/1, or higher or lower. It is also possible to administer more 1-amphetamine than d-amphetamine earlier in the day or the same amounts of each (e.g., in racemic form), as long as the increased 1-ratio later in the day of this invention is achieved. Similarly, the doses of each isomer can be administered in the most varied of ways, e.g., each can be administered separately, together in a single formulation, concurrently, sequentially. Thus, in a preferred embodiment, a d/l ratio greater than one can be achieved in the morning by administering, e.g., substantially only d-isomer, or e.g., a dose of Adderall® mediate release tablets (wherein the d/l ratio is 3/1), or a dose of Adderall XR® (d/l is 3/1 and a second 3/1 pulsed dose is released later in the day), or any other dose of d/l greater than one; and this can be followed by at least one more dose after the morning wherein the ratio of l/d is higher than it was in the morning dose(s) as described above, wherein such later dose is in immediate release form (e.g., over a period of about 30 - about 120 minutes) or extended, sustained or otherwise controlled release form, e.g., to achieve a first order or other release profile over a period of, e.g., 2 to 8 or more hours, depending on the timing of the dose, age of the patient (e.g., an adult desiring later enhanced attentiveness or a child), and other conventional considerations. Of course, the morning dose can also be formulated in various extended, sustained or otherwise controlled release forms. The released relative amounts of d- and l- amphetamine varying during or between time periods also encompass a continuous shift in d/l ratio, e.g., achieved by two different sustained release profiles provided from two different compositional portions administered at the same time (unitarily or separately) or different times; including, for example, having a continuously increasing 1/d ratio from one period to the next, or within any one or two or more periods, or throughout the whole day. A constant d/l ratio may be also be present during a period or from one period to the next, as long as said periods are followed by a later period wherein the 1/d ratio is increased.

Among the many other administration regimens which are suitable for this invention are included those wherein the l-isomer is administered in a fashion such that: its plasma level is essentially constant throughout the day, e.g., in a smooth, sustained release fashion; or it achieves effective plasma levels before or after achievement of at least one effective plasma level of the d-isomer; or its plasma level is continuous or sustained throughout the day while the d-isomer is pulsed or otherwise administered in any of a wide variety of ways, including but not limited to immediate-release or the pulsing modes used in Adderall XR^{®}, or USP 6,322,819, WO 00/2305, USP 6,605,300; or it is administered simultaneously with the d-isomer several times throughout a day; or in various other regimens and variations, or combinations thereof. In all instances, the molar ratio of the two isomers at any given time can remain constant or can vary in either direction consistent with the foregoing, e.g., different ratios can be administered in different pulses. Either of the isomers can be used in free base form, or preferably the 1-isomer, and the other in salt form, or both can be salts or free base, or a mixture of salt and free base forms can be used, etc.

Various other aspects of this invention include the following.

More of the 1-isomer can be administered than currently in Adderall XR^{®} and/or in Adderall^{®} immediate-release tablets in a given day and/or in at least one dose; e.g., where two or three doses are pulsed or otherwise administered per day (e.g., 1-6 hours apart, preferably 2-4 hours apart, as described in, e.g., WO 00/23055), additional l-isomer can be administered separately or within one or more of the administered doses such that the total daily amount of l-isomer is increased; the amount of l-isomer in each of the usual Adderall XR^{®} or immediate-release tablet doses or pulses can be increased by including additional l-isomer in each component of the dosage form. (The weight ratio of d- to l-isomer in these Adderall^{®} products is precisely known from the published amounts of each amphetamine salt in Adderall^{®}, e.g., as in the package insert for Adderall^{®}, immediate release.)

Ratios of l- to d-isomer in at least one of the daily unit doses (e.g., pulses - in extended release modes or tablets/capsules in immediate-release modes) can be increased in accordance with the foregoing.

Different ratios can be used in each of the extended release (e.g., pulse) doses and/or separately administered unit doses in various regimens, including, where two separately administered doses or two extended release pulses are administered: (a) the first dose having amounts of 1 and d-isomers in a molar ratio of 1/3 and the subsequent dose having a ratio of 3/1; or (b) each dose having a ratio of l- and d-isomers of 3:1 or 1:3 followed by another dose of essentially only l-isomer or a ratio of l- and d-isomers of 1:1, or (c) the first dose administered in the early part of the day having an l to d isomer ratio of 1:3 or contains only d isomer, and the subsequent dose having an l to d isomer ratio enhanced in l as described above, e.g., 2:1, 1:1, or contains l isomer only; or, in all these cases, various other l-enhanced relative amounts such as those mentioned above.

Where 3 doses are involved (immediate-release, pulsed, controlled etc.), for example, in early morning (e.g., 7:00 or 8:00 am), mid day (e.g., around noon, e.g., 11:00 am - 1:00 pm) and late afternoon (e.g., around 4:00 - 5:00 pm), the following exemplary molar ratios of d- to l- isomers can be used in each of the three doses in various regimens:
3:1, 3:1, 0:1;
1:0; 1:1; 0:1;
3:1, 1:1, 1:3;
3:1,0:1,0:1;
3:1, 1:1, 1:1;
3:1, 1:3, 1:3;
or the first dose has an l to d isomer ratio of 1:3 or contains only d isomer, and the second and third doses each independently have an enhanced l to d isomer ratio as described above, e.g., 2:1, 1:1, etc., or contain 1 isomer only; as well as a wide variety of other variants, again where the features of the invention are met. As for any multiple dose embodiment of this invention, the doses, here three, can be delivered once in a single staged-release capsule or individually at three different times. See the references above.

Additionally, as long as the 1/d isomer ratio increases later in the day in accordance with the foregoing, many other variants are possible. For example, the preceding periods can have any profile of d/l isomer, including administration regimens where 1 isomer is administered at an increased amount in an earlier period either at an increased l/d ratio or even alone prior to an increase in d/l isomer ratio. Also, in one or more of the extended unit doses (e.g., pulsed doses) or separately administered units, e.g., immediate-release doses, an amount of 1-isomer alone (e.g., 100%, 95%, 90%, 80% etc. enantiomerically pure) can be administered before the combination dose of 1-and d-isomers is administered; or the "pre-dose" of l-isomer can comprise essentially only the 1-isomer or can comprise the 1-isomer and the d-isomer with a ratio of 1- to d-isomer enhanced in 1, as mentioned above, e.g., a ratio greater than 1/3 (1/d), preferably greater than 1:1. The 1-isomer can be pre-administered typically at least one half hour before the subsequent dose, preferably one or more hours before the subsequent dose, e.g., 1 or 2, etc., hours before. Alternatively, the d-isomer can be analogously pre-administered as long as the features of the invention are met. Additional dosing of 1-isomer can also occur early in the day, e.g., before or after the first/last dose of 1-and/or d-isomer in accordance with any of the foregoing. Moreover, in any of the regimens of the invention, the isomers, preferably the 1-isomers, can be administered in free base form wherein they will be more rapidly available in plasma. The isomers, preferably the 1-isomer, in free base (preferably) or salt form, can also be applied on the outside of a capsule or tablet or beads (or other units) contained within a capsule or tablet, etc., where they/it will be available for essentially immediate release upon contact with bodily fluid.

Without wishing to be bound by theory, the beneficial effects of the 1-isomer in this invention are due at least in part to its greater noradrenergic/cortical component than that of the d-isomer, which is believed to have a greater dopaminergic/striatal component.

The foregoing regimens are merely illustrative of the various regimens which can be employed.

This invention also relates to pharmaceutical compositions containing both d-and l-amphetamines according to claim 1 wherein the amount of 1-amphetamine is greater than the amount of d-amphetamine, e.g., molar ratios of l/d of 2/1, 3/1,4/1, 5/1, etc., in certain aspects, not including racemic mixtures or mixtures that contain 48-52 mole % of l isomer of the total amount of amphetamine components. In this regard, this invention also provides compositions containing amphetamines, according to claim 1 in free base form and/or in salt form, in each case comprising a mixture of both d- and l-enantiomers, wherein the molar ratio of the amount of l-amphetamine to d-amphetamine is greater than 1/3, e.g., 1.1/3, 1.5/3, 1.7/3, 1.9/3, 2/3, (2.2, 2.4, 2.6 3,1/1, 4/3, 5/3, 2/1, 7/3, 8/3, 3/1, 10/3,. Ratios between all these exemplary embodiments as well as greater than and less than them while still within the invention, all are included. (The term "ratio" as used herein (above and below) refers always to the molar ratio.)

The combinations and methods of this invention are effective in treating the clinical symptoms of ADHD in children, adolescents, and adults. The administration of enhanced amounts of 1-isomer as described herein has the additional advantages of maintaining attentiveness later in the day, causing lower side effects, e.g., sleep deterioration, etc., especially for preferred embodiments of the invention wherein, as a result, the total d-isomer dose later in the day is lessened from that of conventional treatments, or wherein a lower dose of 1-isomer is administered over the day, especially later in the day to achieve an attentiveness effect (e.g., as measured by the conventional end point of executive functioning / operating (time management, organization, hierarchical thinking, etc.) than would be required to achieve the same effect by d-isomer, thereby in some cases lowering both total amphetamine dose and d-isomer dose. This is especially useful in adolescents and adults where evening attentiveness is useful.

In one aspect of this invention then, the total daily amount of d-amphetamine administered will be lowered from current regimens. One of the results of this aspect will be a lowering of side effects attributable to the d-isomer, e.g., compared to current treatments, including those side effects which also occur with the 1-isomer, but to a lesser extent, e.g., adverse sleep effects. (Side effects of current products include insomnia, sleep disorder, sleep deficit, latency to fall asleep, possible phase shifts in sleep patterns with preference to sleep later in the evening and then to get up later in the morning, anorexia effect, weight loss, food intake effects, and/or abuse potential, and also psychosis, minor skin rashes). Also part of this invention are administration regimens where the amount of d-amphetamine is lower than heretofore but the total amount of amphetamines administered per day is not necessarily lower, e.g., wherein the total amount of l- and d-amphetamines is greater than the total amounts heretofore administered.

The release profiles of amphetamine can be accomplished routinely with a wide variety of conventional formulations, e.g., with structures such as solids having an essentially homogeneous composition or multiple layers, beads, matrices, materials which provide osmotically driven delivery, compartmental delivery forms (e.g., transdermal patches, osmotic forms), and various combinations thereof, e.g., layers on beads, different bead compositions and configurations mixed together in a capsule or separately provided, different ratios of isomers in different compartments, e.g., of a patch, device, composition, any of which can be formulated for immediate, extended, delayed, release.

Formulations for achieving the foregoing dosing regimens are conventional. These can use immediate, controlled, sustained, extended, pulsatile technologies, alone or in combination to achieve the desired regimens. Examples of potential formulations and preparations are contained, for example, in the Handbook of Pharmaceutical Excipents, American Pharmaceutical Association (current edition). Pharmaceutical Dosage Forms: Tablets (Lieberman, Lachman and Schwarts, editors) current edition, published by Marcel Dekker, Inc., as well as Remington's Pharmaceutical Sciences (Arthur Osol, editor, 1553-1593 (current edition)). Where pulses in an extended release capsule or tablet are to be employed, the methods and formulations described in WO 00/23055 can be used, including conventional pulsing techniques such as enteric or other coatings, osmotic systems, and many others. Where smoother release profiles are to be achieved, conventional sustained or controlled release methods can be used; see, e.g., in R.K. Chang and J.R. Robinson, chapter 4: "Sustained Drug Release from Tablets and Particles Through Coating," in Pharmaceutical Dosage Forms: Tablets, volume 3, edited by H.A. Lieberman, L. Lachman, and J.B. Schwartz, Marcel Dekker, Inc., 1991; R.J. Campbell and G.L. Sackett, chapter 3: "Film coating," in Pharmaceutical Unit Operations: Coating, edited by K.E. Avis, A.J. Shukla, and R.K. Chang, Interpharm Press, Inc., 1999.

The amphetamine active ingredients themselves are per se conventional in either the form of isolated isomer, various mixtures of isomers, including racemic, and in various free base and salt forms, including all pharmaceutically acceptable salts, including those mentioned herein. All such isomeric forms are commercially available or routinely preparable, e.g., from racemic mixtures by conventional resolution methods, including diastereomeric addition salts, chromatographic methods, preferably HPLC methods, enzymatic methods, etc. Relative amounts of isomers in a given mixture will vary in accordance with the regimen desired and can vary from racemic to 100% of either isomer and all relative amounts in between.

For example, the amphetamines can be provided in the form of beads, e.g., having a core which is optionally coated with a coating which allows the release of the amphetamine salts there through immediately or over time, such as a pharmaceutically acceptable water-insoluble or water soluble film former alone or with a dissolution regulating agent etc.. See, e.g., USP 4,728,512. A biphasic or multiphasic release profile can be achieved by combining the immediate-release beads with delayed, sustained or other controlled release beads or by providing various extended release beads with differing release profiles.

Beads can be prepared by coating conventional drug-containing cores with a water-insoluble polymer, or a combination of water-insoluble polymers, or a combination of water-insoluble and water-soluble polymers. This may be a combination of layers, or a combination of polymers in a single coating. The resultant beads (or tiny tablets) can then be placed in a capsule. Other than beads in a capsule shell, tablets in a capsule shell (e.g., one or more immediate-release tablet and one or more delayed, sustained release tablet in a capsule shell) also can be used to attain the desired release profile.

Various polymeric materials can be used to achieve the desired type of pattern of release, e.g., immediate, sustained, delayed release. For example, a multiple dosage form (e.g., as discussed below) can deliver rapid and complete dosages of pharmaceutically active amphetamine base or salts to a recipient multiple times over a period of hours with a single oral administration, if it is desired to combine dosages in a single unit; additionally, doses with sustained or delayed release function can be combined with each other or with doses having immediate release functionality. See e.g., USP 6,322,819.

Examples of possible bead constructions include the following:
- Sugar core, coated with amphetamine, coated with polymer,
- Sugar core, coated with amphetamine, coated with mix of amphetamine and polymer, coated with polymer,
- Sugar core, coated with amphetamine, coated with relatively high concentration mix of amphetamine and polymer, coated with weaker concentration mix of amphetamine and polymer, coated with polymer,
- Bead containing amphetamine, coated with polymer,
- Bead containing amphetamine, coated with mix of amphetamine and polymer, coated with polymer,
- Bead containing amphetamine, coated with relatively high concentration mix of amphetamine and polymer, coated with weaker concentration mix of amphetamine and polymer, coated with polymer, and
- Tablet or capsule containing multiple types of beads as described above having differing timing of release of amphetamine and/or different rates of release of amphetamine.

In addition to layered beads, matrix beads can also be used, i.e., not having any needed layers to achieve sustained or delayed release. The components used in such matrices are chosen from conventional sustained release or delayed release polymers.

Details of using the foregoing constructs and others to achieve a desired release profile as discussed above are fully conventional and can be determined by those of skill in the art with at most a few routine parametric experiments, and conventional adjustments, e.g., involving identities of polymers and mixtures thereof, relative amounts of components, coating thicknesses, bead diameters, number of layers and compositions thereof. Thus, for example, for a given construct, dissolution profiles as described herein can be determined and in vivo plasma profiles measured. Fully conventional formulation and dissolution profile adjustments can be made routinely. The formulations having the release profiles described herein will produce plasma levels having the desired properties of increased l/d ratio later in the day in accordance with this invention. These plasma profiles will be correlated with the release profiles in view of the usual factors, e.g., in vivo dissolution and absorption properties, amphetamine isomer half-lives (the 1-isomer being slightly longer), etc., which correlation is well understood for amphetamines and is routinely determinable for any given amphetamine combination of this invention.

Suitable materials which can be used in the sustained release formulations of this invention are well known and include polyvinyl acetate, cellulose acetate, cellulose acetate butyrate, cellulose acetate propionate, ethyl cellulose, fatty acids and their esters, alkyl alcohols, waxes, zein (prolamine from corn), and aqueous polymeric dispersions such as Eudragit RS and RL30D, Eudragit NE30D, Aquacoat, Surelease, Kollicoat SR30D, and cellulose acetate latex.

Methods of manufacturing cores include:
a. Extrusion-Spheronization - the drug(s) and other additives are granulated with the addition of a binder solution. The wet mass is passed through an extruder equipped with a certain size screen. The extrudates are spheronized in a marumerizer. The resulting pellets are dried and sieved for further applications.
b. High-Shear Granulation - Drug(s) and other additives are dry-mixed and then the mixture is wetted by addition of a binder solution in a high shear-granulator/mixer. The granules are kneaded after wetting by the combined action of mixing and milling. The resulting granules or pellets are dried and sieved for further applications.
c. Solution or Suspension Layering- A drug(s) solution or dispersion with or without a binder is sprayed onto starting seeds with a certain particle size in a fluidized bed processor or other suitable equipment. The drug thus is coated on the surface of the starting seeds. The drug-loaded pellets are dried for further applications.

For purposes of the present invention, the core particles, preferably, have a diameter in the range of about 50-1500 microns (micrometers); more preferably 100-800 microns. These particles can then be coated in a fluidized bed apparatus with an alternating sequence of selected coating layers.

Bead forms can be incorporated for example into hard gelatin capsules, either with additional excipients, or alone. Typical excipients to be added to a capsule formulation include fillers such as microcrystalline cellulose; soy polysaccharides, calcium phosphate dihydrate, calcium sulfate, lactose, sucrose, sorbitol, or any other inert filler. In addition, there can be flow aids such as fumed silicon dioxide, silica gel, magnesium stearate, calcium stearate or any other material imparting flow to powders. A lubricant can further be added if necessary by using, for example, polyethylene glycol, leucine, glyceryl behenate, magnesium stearate or calcium stearate.

The composition may also be incorporated into a tablet, in particular by incorporation into a tablet matrix, which rapidly disperses the particles after ingestion. In order to incorporate these particles into such a tablet, a filler/binder must be added to a tablet that can accept the particles, but will not allow their destruction during the tableting process. Materials that are suitable for this purpose include microcrystalline cellulose (AVICEL.RTM.), soy polysaccharide (EMCOSOY.RTM.), pre-gelatinized starches (STARCH.RTM. 1500, NATIONAL.RTM. 1551), and polyethylene glycols (CARBOWAX.RTM.). The materials are preferably present in the range of 5-75% (w/w), with a more preferred range of 25-50% (w/w).

In addition, disintegrants are optionally added in order to disperse the beads once the tablet is ingested. Suitable disintegrants include : cross-linked sodium carboxymethyl cellulose (AC-DI-SOL.RTM.), sodium starch glycolate (EXPLOTAB.RTM., PRIMOJEL.RTM.), and cross-linked polyvinylpolypyrrolidone (Plasone-XL). These materials are preferably present in the rate of 3-15% (w/w), with a more preferred range of 5-10% (w/w).

Lubricants are also optionally added to assure proper tableting, and these can include: magnesium stearate, calcium stearate, stearic acid, polyethylene glycol, leucine, glyceryl behanate, and hydrogenated vegetable oil. These lubricants are preferably present in amounts from 0.1-10% (w/w), with a more preferred range of 0.3-3.0% (w/w).

Tablets are formed, for example, as follows. The particles are introduced into a blender along with AVICEL.RTM., disintegrants and lubricant, mixed for a set number of minutes to provide a homogeneous blend which is then put in the hopper of a tablet press with which tablets are compressed. The compression force used is adequate to form a tablet; however, not enough to fracture the beads or coatings.

Where immediate release is not desired, for example, various enteric materials, e.g., cellulose acetate phthalate, hydroxypropyl methylcellulose phthalate, polyvinyl acetate phthalate, and the EUDRAGIT.RTM. acrylic polymers, can be used as gastroresistant, enterosoluble coatings for drug release in the intestine when desired. The enteric materials, which are soluble at higher pH values, are frequently used for colon-specific delivery systems and are entirely conventionally employable in the SR systems of this invention. The enteric polymers used in this invention can also be modified conventionally by mixing with other known coating products that are not pH sensitive. Examples of such coating products include the neutral methacrylic acid esters with a small portion of trimethylammonioethyl methacrylate chloride, sold currently under the trade names EUDRAGIT.RTM. and EUDRAGIT.RTM. RL; a neutral ester dispersion without any functional groups, sold under the trade names EUDRAGIT.RTM. NE30D and EUDRAGIT.RTM. NE30; and other pH independent coating products.

A conventional protective coating layer may also be applied immediately outside the core, either a drug-containing matrix core or a drug-layered core, by conventional coating techniques such as pan coating or fluid bed coating using solutions of polymers in water or suitable organic solvents or by using aqueous polymer dispersions. Suitable materials for the protective layer include cellulose derivatives such as hydroxyethyl cellulose, hydroxypropyl cellulose, hydroxypropyl methylcellulose, polyvinylpyrrolidone, polyvinylpyrrolidone/vinyl acetate copolymer, ethyl cellulose aqueous dispersions (AQUACOAT.RTM., SURELEASE.RTM.), EUDRAGIT.RTM. RL 30D, OPADRY.RTM. Typical coating levels are from 1 to 6%, preferably 2-4% (w/w).

An overcoating layer can further optionally be applied to the composition of the present invention. OPADRY.RTM., OPADRY II.RTM. (Colorcon) and corresponding color and colorless grades from Colorcon can be used to protect the pellets from being tacky and provide colors to the product. Typical levels of protective or color coating are from 1 to 6%, preferably 2-3% (w/w). Many ingredients can be incorporated into the overcoating formula, for example to provide a quicker (immediate) release, such as plasticizers: acetyltriethyl citrate, triethyl citrate, acetyltributyl citrate, dibutylsebacate, triacetin, polyethylene glycols, propylene glycol and the others; lubricants: talc, colloidal silica dioxide, magnesium stearate, calcium stearate, titanium dioxide, magnesium silicate.

Optional modifying components of a protective layer which can be used over the enteric or other coatings include a water penetration barrier layer (semi-permeable polymer) which can be successively coated after the enteric or other coating to reduce the water penetration rate through the enteric coating layer and thus increase the lag time of the drug release. Sustained-release coatings commonly known to one skilled in the art can be used for this purpose by conventional coating techniques such as pan coating or fluid bed coating using solutions of polymers in water or suitable organic solvents or by using aqueous polymer dispersions. For example, the following materials can be used: cellulose acetate, cellulose acetate butyrate, cellulose acetate propionate, ethyl cellulose, fatty acids and their esters, waxes, zein, and aqueous polymer dispersions such as EUDRAGIT.RTM. RS and RL 30D, EUDRAGIT.RTM. NE 30D, AQUACOAT.RTM., SURELEASE.RTM., cellulose acetate latex. The combination of the above polymers and hydrophilic polymers such as hydroxyethyl cellulose, hydroxypropyl cellulose (KLUCEL.RTM., Hercules Corp.), hydroxypropyl methylcellulose (METHOCEL.RTM., Dow Chemical Corp.), polyvinylpyrrolidone can also be used.

Principles of sustained release formulation technology applicable to this invention, including the exemplary modes mentioned herein, are disclosed, e.g., in R.K. Chang and J.R. Robinson, chapter 4: "Sustained Drug Release from Tablets and Particles Through Coating," in Pharmaceutical Dosage Forms: Tablets, volume 3, edited by H.A. Lieberman, L. Lachman, and J.B. Schwartz, Marcel Dekker, Inc., 1991; R.J. Campbell and G.L. Sackett, chapter 3: "Film coating," in Pharmaceutical Unit Operations: Coating, edited by K.E. Avis, A.J. Shukla, and R.K. Chang, Interpharm Press, Inc., 1999.

Where it is desired to achieve a pulsed delivery, e.g., a first immediate or delayed or sustained release followed by a second such release later in time, the technique of USP 6,322,819 may be used. In this aspect, the amphetamine base or salts can be provided within or as a part of a core seed around which the enteric coating is applied. Alternatively, a core seed can be coated with one or more layers of amphetamines. In this technique, a delayed immediate release drug delivery can also be accomplished by coating the drug with an enteric coating. With respect to this embodiment, the amphetamines can be provided within or as a part of a core seed, during the core seed manufacturing process. Alternatively, a core seed can be coated with one or more layers of amphetamine.

In one aspect of this embodiment, a semi-permeable polymer, which may comprise a low water-permeable pH-insensitive polymer, is layered onto the outer surface of the enteric layer, in order to obtain prolonged delayed release time. This semi-permeable polymer coating controls the erosion of the pH-sensitive enteric polymer in an alkaline pH environment in which a pH-sensitive polymer will dissolve rapidly. Another pH-sensitive layer may be applied onto the surface of a low water-permeability layer to further delay the release time. In addition to a protective layer, the composition may comprise an acid which is incorporated into the pharmaceutical active layer or coated onto the surface of the active layer to reduce the pH value of the environment around the enteric polymer layer. The acid layer may also be applied on the outer layer of the pH-sensitive enteric polymer layer, followed by a layer of low water-permeability polymer. The release of the active thus may be delayed and the dissolution rate may be increased in an alkaline environment.

Pulsatile delivery can also be achieved by combination materials with an immediate release component surrounding a compressed core coated which ruptures after a period of exposure to gastric fluids, see USP 6,183,780. Various other pulsatile release forms are disclosed in US Pat. No. 6,340,476.

The formulations of the invention may moreover be in the form of a tablet, e.g., as disclosed in USP 6,384,020, e.g., comprising lactitol (4-O-(-D-Galactopyranosyl-D-glucitol monohydrate), and may further comprise bulking agents, disintegrating agents, antiadherants, glidants, lubricants, colorants, binding agents.

The bulking agents employed herein can be microcrystalline cellulose, for example, AVICEL.RTM. (FMC Corp.) or EMCOCEL.RTM. (Mendell Inc.); dicalcium phosphate, for example, EMCOMPRESS.RTM. (Mendell Inc.); calcium sulfate, for example, COMPACTROL.RTM. (Mendell Inc.); and starches, for example, STARCH 1500. As disintegrating agents there may be employed herein microcrystalline cellulose, starches, crospovidone, for example, POLYPLASDONE XL.RTM. (International Specialty Products); sodium starch glycolate, for example, EXPLOTAB.RTM. (Mendell Inc.); and croscarmellose sodium, for example, AC-DI-SOL.RTM. (FMC Corp.). Antiadherants and glidants employed herein can include talc, corn starch, silicon dioxide, sodium lauryl sulfate, and metallic stearates. Lubricants employed herein can be magnesium stearate, calcium stearate, sodium stearate, stearic acid, sodium stearyl fumarate, sterotex, talc, colloidal silica dioxide, waxes and the like. Binding agents employed herein can include polyvinyl pyrrolidone, starch, methyl cellulose, hydroxypropyl methylcellulose, carboxymethyl cellulose, and the like. Tablets can be prepared using methods known in the art including a wet granulation method or direct compression.

The present compositions can also have a pH-independent or a minimized pH-dependent dissolution profile, e.g., as disclosed in USP 6,287,599. Such a composition may include at least one pharmaceutically active agent that has a pH dependent solubility profile, at least one non-pH-dependent sustained release agent, and at least one pH-dependent agent that increases the dissolution rate of the at least one pharmaceutically active agent at a pH, e.g., in excess of 5.5.

Non-pH-dependent sustained release agents which may be included in such a composition include, but are not limited to, ethylcellulose, cellulose acetate, vinyl acetate/vinyl chloride copolymers, acrylate/methacrylate copolymers, polyethylene oxide, hydroxypropyl methylcellulose, carrageenan, alginic acid and salts thereof, hydroxyethyl cellulose, hydroxypropyl cellulose, karaya gum, acacia gum, tragacanth gum, locust bean gum, guar gum, sodium carboxymethyl cellulose, methyl cellulose, beeswax, carnauba wax, cetyl alcohol, hydrogenated vegetable oils, and stearyl alcohol. In general, the at least one non-pH-dependent sustained release agent is present in the composition in an amount of from 5 wt. % to 50 wt. %, preferably from 10 wt. % to 30 wt. %.

pH-dependent agents that increase the rate of release of the at least one pharmaceutically active agent from the tablet at a pH in excess of 5.5 include polymers that swell at a pH in excess of 5.5, and enteric agents, and/or agents that increase the solubility of the at least one pharmaceutically active agent at a pH greater than 5.5, by maintaining an acidic microenvironment in the tablet, e.g., an organic acid. The at least one pH-dependent agent is present in the composition in an amount of from 0.5 wt. % to 40 wt. %, preferably from wt. % to 20 wt. %. Polymers that swell at a pH in excess of 5.5 include acrylic acid copolymers, sodium alginate, carrageenan, alginic acid, pectin, and sodium carboxymethyl cellulose. Enteric agents include all those above, e.g., cellulose acetate phthalate, hydroxypropyl methylcellulose phthalate, polyvinyl acetate phthalate, methacrylic acid copolymers, cellulose acetate trimellitate, hydroxypropyl methylcellulose acetate, succinate, shellac, and zein. Agents that increase the solubility of the at least one pharmaceutically active agent at a pH greater than 5.5 include organic acids. Such organic acids maintain an acidic microenvironment in the tablet, and include citric acid, fumaric acid, tartaric acid, adipic acid, glucono delta-lactone, and malic acid. The pH-independent or composition may further include other materials such as bulking agents, disintegrating agents, anti-adherants and glidants, lubricants, binding agents etc. Disintegrating agents which may be included in the composition include microcrystalline cellulose, starches, crospovidone (e.g., Polyplasdone XL, International Specialty Products.), sodium starch glycolate (Explotab, Mendell Inc.), and crosscarmellose sodium (e.g., Ac-Di-Sol, FMC Corp.). The disintegrating agent may be present in the composition in an amount of from 0.5 wt. % to 30 wt %, preferably from 1 wt. % to 15 wt. %.

Antiadherants and glidants which may be employed in the composition include talc, corn starch, silicon dioxide, sodium lauryl sulfate, and metallic stearates. The antiadherant or glidant may be present in the composition in an amount of from 0.2 wt. % to 15 wt. %, preferably from 0.5 wt. % to 5 wt. %. Lubricants which may be employed in the composition include magnesium stearate, calcium stearate, sodium stearate, stearic acid, sodium stearyl fumarate, hydrogenated cotton seed oil (sterotex), talc, and waxes, including beeswax, camuba wax, cetyl alcohol, glyceryl stearate, glyceryl palmitate, glyceryl behenate, hydrogenated vegetable oils, and stearyl alcohol. The lubricant may be present in an amount of from 0.2 wt. % to 20 wt. %, preferably from 0.5 wt. % to 5 wt. %. Binding agents which may be employed include polyvinyl pyrrolidone, starch, methylcellulose, hydroxypropyl methylcellulose, carboxymethyl cellulose, sucrose solution, dextrose solution, acacia, tragacanth and locust bean gum. The binding agent may be present in the composition in an amount of from 0.2 wt. % to 10 wt. %, preferably from 0.5 wt. % to 5 wt. %.

The compositions of the present invention may be made by methods as above, including a direct compression method, or by a wet granulation method. In the direct compression method, the at least one pharmaceutically active agent and other ingredients may be sieved through a stainless steel screen, such as a 40 mesh steel screen. The sieved materials then are charged to a suitable blender, and blended, e.g., for 10 minutes with an intensifier bar on for 3 minutes. The blend then can be compressed into tablets on a rotary press using appropriate tooling. The compressed tablets may be coated, if desired.

In the wet granulation method, the at least one pharmaceutically active agent and other ingredients may be granulated with a granulating fluid (e.g., isopropyl alcohol, ethyl alcohol, and water) in a planetary mixer, high shear mixer, or fluidized bed granulator. Binding agents may be contained in the granulating fluid, or may be in the dry mix. The wet granules may be dried in an oven or fluidized bed dryer, and then sieved through a suitable screen to obtain free flowing granules. The resulting granules may be blended with a suitable lubricant and glidant, and the lubricated granules are compressed into tablets on a rotary press using appropriate tooling. If desired, a coating can be applied onto the compressed tablets.

Conventional osmotic delivery systems may also be used. Such systems are disclosed in, e.g., US Patent Nos. 5,308,348; 5,260,069; 5,232,705; 4,871,549; 5,156,850; 5,312,388; 6,110,498 and 6,284,276.

In the preferred embodiment, a composition of this invention is administered to a patient orally, e.g., in the form of a tablet, capsule, liquid, gel, inhalant. Agents used in this invention can also be administered by injection which includes intravenous, intraarticular, intramuscular, subcutaneous and parenteral injections, as well as use of infusion techniques. Dermal administration may include topical application or transdermal administration. Agents may be present in association with one or more non-toxic pharmaceutically acceptable carriers and if desired other active ingredients, e.g., vitamins, other active agents for the involved indication.

Compositions intended for oral use may be prepared according to any suitable method known to the art for the manufacture of pharmaceutical compositions. Such compositions may contain one or more agents including diluents, sweetening agents, flavoring agents, coloring agents and preserving agents in order to provide palatable preparations.

Tablets contain the active ingredient(s) in admixture with non-toxic pharmaceutically acceptable excipients which are suitable for the manufacture of tablets. These excipients may be, for example, inert diluents, such as calcium carbonate, sodium carbonate, lactose, calcium phosphate, or sodium phosphate; granulating and disintegrating agents, for example, corn starch, or alginic acid; and binding agents, for example magnesium stearate, stearic acid or talc. The tablets may be uncoated or they may be coated by known techniques to delay disintegration and adsorption in the gastrointestinal tract and thereby provide a sustained action over a longer period. For example, a time delay material such as glyceryl monosterate or glycerol distearate may be employed. These compounds may also be prepared in solid, rapidly released form.

Formulations for oral use may also be prepared as hard gelatin capsules wherein the active ingredient is mixed with an inert solid diluent, for example, calcium carbonate, calcium phosphate or kaolin, or as soft gelatin capsules wherein the active ingredient is mixed with water or an oil medium, for example peanut oil, liquid paraffin or olive oil.

Where applicable, the present invention also involves useful forms of active agents as disclosed herein, such as pharmaceutically acceptable salts Pharmaceutically acceptable salts include those obtained by reacting the main compound, functioning as a base, with an inorganic or organic acid to form a salt, for example, salts of hydrochloric acid, sulfuric acid, phosphoric acid, methane sulfonic acid, camphor sulfonic acid, oxalic acid, maleic acid, succinic acid, citric acid. Pharmaceutically acceptable salts also include those in which the main compound functions as an acid and is reacted with an appropriate base to form, e.g., sodium, potassium, calcium, magnesium, ammonium, choline salts, etc.

Various liquid oral dosage forms can also be used for administering active agents of the invention, including aqueous and non-aqueous solutions, emulsions, suspensions, syrups, and elixirs. Such dosage forms can also contain suitable inert diluents known in the art such as water and suitable excipients known in the art such as preservatives, wetting agents, sweeteners, flavorants, as well as agents for emulsifying and/or suspending. Agents used in the present invention may be injected, for example, intravenously, in the form of an isotonic sterile solution.

Suppositories for rectal administration can be prepared by mixing the compound with a suitable excipient such as cocoa butter, salicylates, and polyethylene glycols. Formulations for vaginal administration can be in the form of a pessary, tampon, cream, gel, paste, foam or spray formula containing, in addition to the active ingredient, such suitable carriers as are known in the art.

For topical administration, there can be used creams, ointments, liniments, lotions, emulsions, suspensions, gels, solutions, pastes, powders, sprays, and drops suitable for administration to the skin, eye, ear, or nose. Topical administration may also involve transdermal administration via means such as transdermal patches.

Aerosol formulations suitable for administering via inhalation also can be made. For example, the agents can be administered by inhalation in the form of a powder (e.g., micronized) or in the form of atomized solutions or suspensions. The aerosol formulation can be placed into a pressurized propellant.

It will be appreciated by those skilled in the art that the particular way of administration will depend on a variety of factors, all of which are routinely considered when administering therapeutics. It will also be understood, however, that the specific dose level for any given patient will depend upon a variety of factors, including the activity of the specific compound employed, the age of the patient, the diet of the patient, time of administration, route of administration, rate of excretion, drug combinations, and the severity of the condition undergoing therapy. It will be further appreciated by one skilled in the art that the optimal course of treatment, i.e., the mode of treatment, the daily number of doses, the number of days of treatment, etc., can be routinely ascertained by those skilled in the art with conventional treatment tests where appropriate.

### Brief Description of the Drawings

Various features and attendant advantages of the present invention will be more fully appreciated as the same becomes better understood when considered in conjunction with the accompanying drawings, which show:
Figure 1 - In vivo DA release caused by d- and 1-amphetamines from striatum using microdialysis.
Figure 2 - In vivo NA release caused by d- and 1-amphetamines from frontal cortex using microdialysis.
Figure 3 - Effects of d- and 1-amphetamines on general activity level.
Figure 4 - Effects of d- and 1-amphetamines on impulsiveness.
Figure 5 - Effects of d- and 1-amphetamines on sustained attention.
Figure 6 - Effects of d- and 1-amphetamines on sustained attention following transformation.
Figure 7 - Effects of d- and 1-amphetamines and combinations on the general activity level.
Figure 8 - Effects of d- and 1-amphetamines and combinations on impulsiveness.
Figure 9 - Effects of d- and 1-amphetamines and combinations on sustained attention.
Figure 10 - Effects of d- and l-amphetamines and combinations on sustained attention after transformation.
Figure 11 - Effects of d- and l-amphetamines and dl-amphetamines on % wakefulness.
Figure 12 - Effects of d- and l-amphetamines and dl-amphetamines on % delays to rapid eye movement sleep.

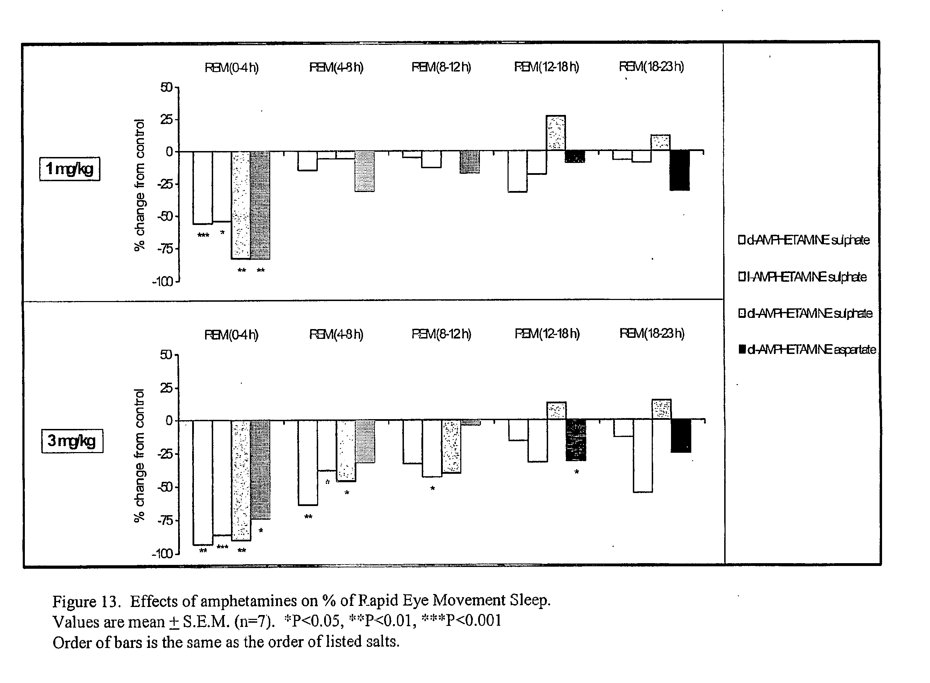

Figure 13 - Effects of d- and l-amphetamines and dl-amphetamines on % of rapid eye movement sleep.
Figure 14 - Effects of d- and l-amphetamines and dl-amphetamines on % delays to slow wave sleep.

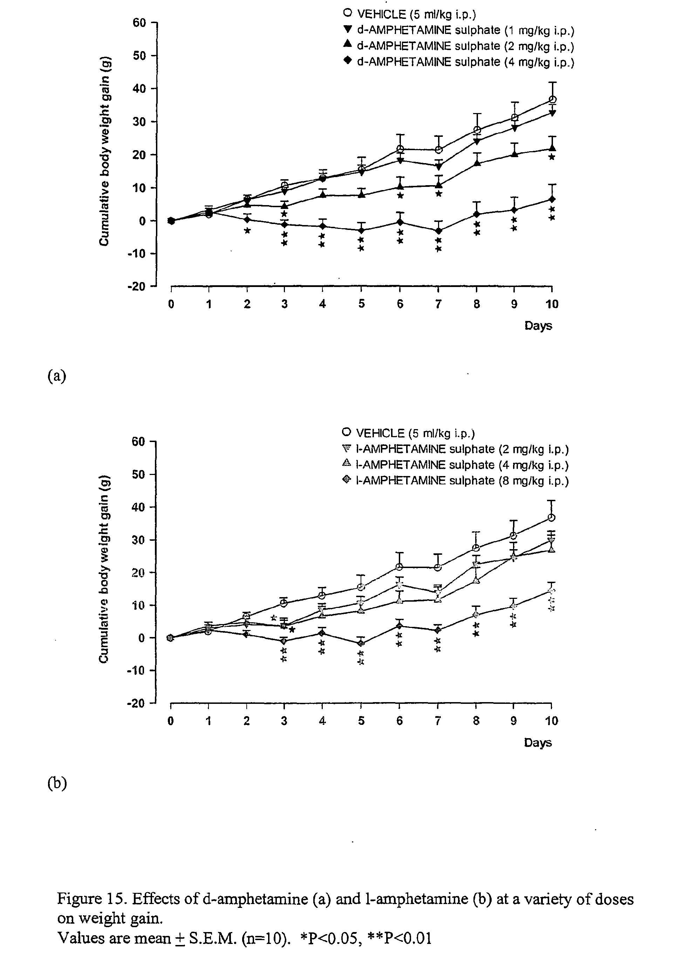

Figure 15 - Effects of d-and l-amphetamines on weight gain.

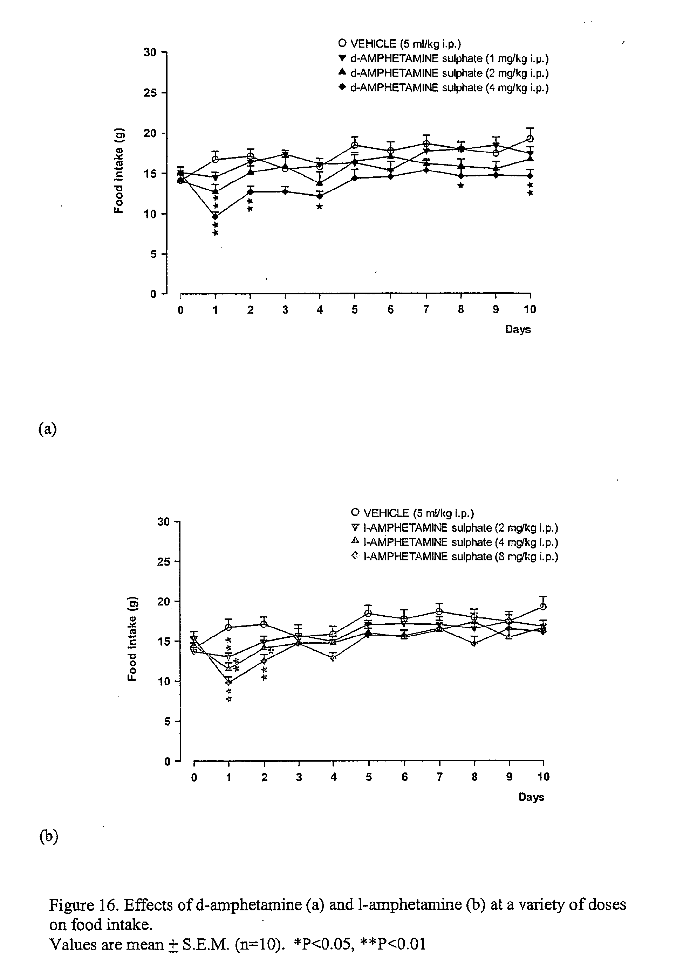

Figure 16 - Effects of d- and l-amphetamines on food intake.

### Examples

Without further elaboration, it is believed that one skilled in the art can, using the preceding description, utilize the present invention to its fullest extent. The following preferred specific embodiments are, therefore, to be construed as merely illustrative.

In the foregoing and in the following examples, all temperatures are set forth uncorrected in degrees Celsius and, all parts and percentages are by weight, unless otherwise indicated.

### Example 1 - Release of Monoamines in vitro

Effects of the amphetamine isomers d-amphetamine and 1-amphetamine on the release of the monoamines: dopamine (DA) and noradrenaline (NA), in rat brain slices, *in vitro,* are evaluated. The DA and NA release is measured in separate brain areas, i.e., in striatum for DA release (evaluating tritiated dopamine) and in the frontal cortex for NA release (evaluating tritiated noradrenaline), using a Brandel system. The brain slices are loaded with the tritiated monoamine (dopamine or noradrenaline) and then superfused to produce basal neurotransmitter release. The slices are also subjected to 2 short periods of electrical stimulation to effect stimulated transmitter release. The effects of d-amphetamine and 1-amphetamine on basal release and electrically stimulated release of tritium are determined. Three concentrations are tested, starting at around 1 µM and titrating up or down, to produce an EC₅₀ value. No positive control is needed as the amphetamine will act as its own control, but there is a vehicle control. The EC₅₀ values for basal release are shown in the following table.

| | DA/Striatum | NA/Frontal cortex |
|---|---|---|
| D-amphetamine | 1.1 (0.6-2.0) µM | 0.5 (0.2-1.3) µM |
| L-amphetamine | 3.9 (3.0-5.2) µM | 0.3 (0.1-0.4) µM |

D-amphetamine is shown to have similar potency in releasing NA from cortex and DA from striatum. L-amphetamine is considerably more potent at releasing NA from cortex than DA from striatum.

### Example 2 - Release of Monoamines in vivo

Release of monoamines is measured *in vivo,* using a technique called microdialysis, which monitors the chemistry of the extracellular space in living tissue, in this case the brain. A microdialysis probe is inserted into the brain such that its tip lies within the brain area of interest. This probe consists of a concentric tube where the perfusion fluid enters through an inner tube, flows to its distal end (the tip which is in the brain region where release is to be measured) and then exits the tube and enters the space between the inner tube and the outer dialysis membrane. This is where the "dialysis" takes place, i.e. the diffusion of molecules between the extracellular fluid and the perfusion fluid. When a physiological salt solution is slowly pumped through the microdialysis probe, the solution equilibrates with the surrounding extracellular tissue fluid. After a while, it will contain a representative proportion of the tissue fluid's molecules. The microdialysate is extracted and analyzed in the laboratory using HPLC.

Using this procedure tests are conducted using a saline control, d-amphetamine sulphate at two concentrations of 1 and 3 mg/kg i.p. and 1-amphetamine hydrochloride at three concentrations of 1, 3 and 9 mg/kg i.p.. Experiments are carried out in male Sprague-Dawley rats, approximately 250-350g body weight. The animals are housed for at least 5 days prior to the study to acclimatise. On day one, surgery is performed, the rats are placed into dialysis 'bins' and the experiment is conducted on day two. The rats have dual probes implanted into the prefrontal cortex and striatum. Dialysate samples are collected every 30 min and a total of 11 samples are collected for each brain region (3 pre-drug and 8 post-drug). The HPLC samples are subsequently run. For determination of DA and NA the microdialysis samples are split (10 µl for DA, 20 µl for NA) and assayed on separate HPLCs. The experiment includes a saline-treated control group and the five groups of the differing doses of the 2 drugs; thus, 6 different treatment groups. Final group sizes of 5-6 rats for the drug-treated groups and 10-12 rats for the saline control group are employed.

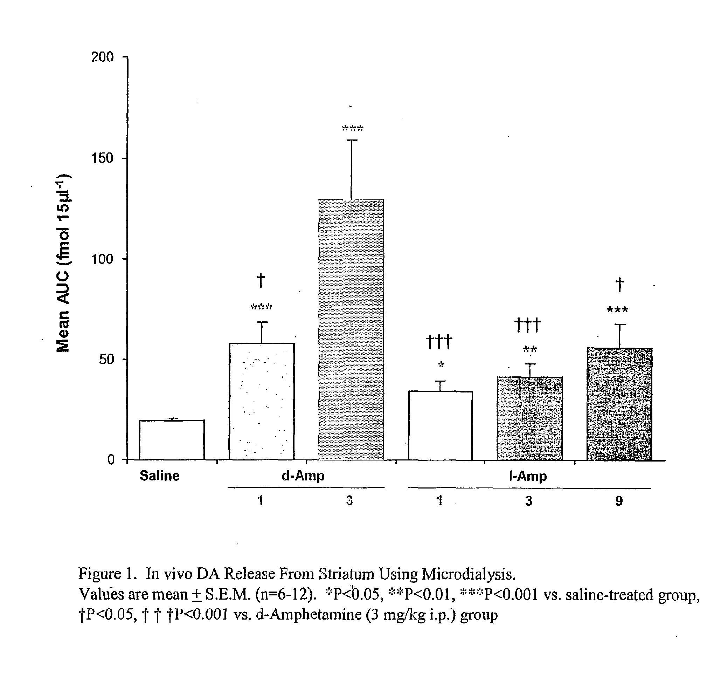

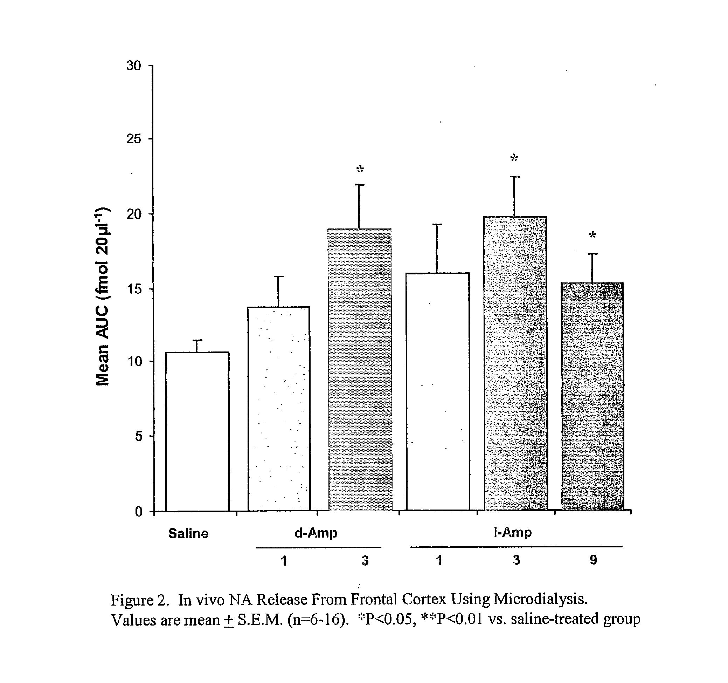

The results for DA release from the striatum, measured as mean AUC (area under the curve of the HPLC) are shown in Figure 1. The data show that D-amphetamine has greater potency than L-amphetamine in releasing DA from the striatum in this model. The results for NA release from the frontal cortex, measured as mean AUC, are shown in Figure 2. The data show that D-amphetamine and L-amphetamine have similar potency in releasing NA from the frontal cortex in this model.

### Example 3 - Behavioural Study of Effects of Amphetamine Isomers

ADHD is currently defined as a cognitive/behavioral developmental disorder where all clinical criteria are behavioral. Inattentiveness, overactivity, and impulsiveness are presently regarded as the main clinical symptoms. The spontaneously hypertensive rat (SHR) is the best validated animal model of ADHD. These rats show hyperactivity, deficits in attention and impulsivity (Sagvolden, 2000; Sagvolden, 2001; Sagvolden, Aase, Johansen, & Farshbaf, 2004a). The control strain is usually the Wistar Kyoto Rat (WKY) as this rat is the progenitor strain and its behavior is closely similar to that of other strains when tested in operant tasks (Sagvolden, 2000; Sagvolden, 2001; Sagvolden et al., 2004a).

### Procedure

*Subjects:* A total number of 32 male rats, 16 spontaneously hypertensive (SHR) and 16 Wistar/Kyoto (WKY), participated in final part of this study. (Initially, 8 extra rats, 6 SHRs and 2 controls, were trained with a view to select two groups of 16 with the greatest difference in baseline measures. However, there was little difference when comparing all the rats with a selected subgroup - they both separated well.) At the start of testing, the rats were -5 wk old and experimentally naïve. Young rats were required, as ADHD primarily is a child and adolescent disorder. The SHRs were obtained from Charles River Italy (SHR/Crl Ico) and the WKYs from Charles River France (WKY/Nico) as other breeding centers were unable to deliver rats at the time we needed them. At the University of Oslo, the rats were housed individually in 41 x 25 x 25 (height) cm transparent cages and had free access to food (BK Standard RM 1 from Beekay Feeds Universal Ltd.).

The rats had access to water at all times during the habituation period. However, after completing the habituation period, the rats were deprived of water for 21 hr a day. The rats received water as reinforcers during the experimental session and had free access to water for 90 min after the experimental session. The temperature in the housing area was ~20°C. The light in the housing area was on from 0700 to 1900 hours. The behavioral training took place between 0900 and 1230 hours seven days a week.

*Behavioral apparatus:* Sixteen Campden Instruments operant chambers were used in the study. The animal working space in eight of the chambers is 25 x 25 x 30 (height) cm and 25 x 25 x 20 (height) cm in the other eight chambers. The 2.8-W house light and a fan producing a low masking noise were on during the entire experimental session.

During training sessions, either one or two retractable levers were used. A 2.8-W cue light was located above each lever. The rats' response consisted of pressing one of the levers with a dead weight of at least 3 g to activate a microswitch. The reinforcers (0.01 ml tap water) were delivered by a liquid dipper located in a small recessed cubicle with a 2.8-W cue light lit when a reinforcer is presented. A 7 x 5 cm transparent plastic lid separated the cubicle from the rat's working space. The rat could easily open the lid with a light push with the nose or paw. Each chamber was ventilated and placed in a sound-resistant outer housing. A computer and an online system (SPIDER, Paul Fray, Ltd., UK) recorded the behavior and scheduled reinforcers (drops of water).

Before the initiation of the study, the rats were randomized and balanced into two groups. Each group of rats consisted of an equal number of SHRs and WKYs. Each rat was assigned to a specific chamber, which it occupied during all sessions. The rat was returned to its living cage after each session. All animals were run seven days a week.

*Response acquisition:* The training period started with a single 30-min habituation session. During the habituation session, the lid between the working space and the reinforcement cubicle was kept open. No lever was present and no cue light above any lever was lit. The house light was on. From this time on the rats were deprived of water for 21 hr a day; this is a moderate, but sufficient deprivation for motivating the animal.

The habituation session was followed by two 30-min magazine training sessions. The lid was taped open, no levers were present, and the house light was on, but the cue lights above the levers were not lit. The computer delivered water on the average every 10 s independent of the rat's behavior (a variable-time schedule).

In the next two sessions, the rat was trained to open the lid to gain access to the water. The lid was not taped open, no levers were present and the lights above the levers were not activated. The house light was on. Each lid opening was followed by a presentation of a single drop of water.

During the subsequent two sessions, lever responding was shaped by the method of successive approximations (Catania, 1998). During the first of these sessions, the rats learned to press the left lever in order to receive a reinforcer after every press. The cue light above the left lever was now lit. The right lever was retracted into the wall and the light above the right lever was not lit. On the second session, the right lever was activated and the left lever retracted. During this session the light above the right lever was activated. The house light was on. This shaping procedure lasted just one session, then the animal had acquired the appropriate behavior. From now on, both levers were present. The light above the levers shifted randomly. The light showed the rat which lever it had to press to receive a reinforcer ("correct lever"). The first four of these sessions lasted for 30 min and the reinforcers were delivered following every correct lever press. Then followed a single session when the reinforcers were delivered according to a 15-s Random-Interval (RI) schedule. The rat received the reinforcer immediately after pressing the correct lever. A concurrent extinction (EXT) schedule was present on the wrong lever.

*Final schedule:* From session 13 on until the study was finished, a 180-s Random-Interval (RI) schedule was in effect on the correct lever. A concurrent extinction (EXT) schedule was present on the wrong lever. A reinforcement schedule is called multiple when two (or more) schedules are run and each of these is signaled. Thus, a multiple Random-Interval/Extinction (mult RI EXT) schedule was applied for testing effects of the drugs in the present study.

During the final multiple Random-Interval/Extinction schedule, reinforcers were delivered on average every 180s. There was neither any external stimulus signaling that a reinforcer was programmed according to the RI schedule, nor any external stimulus signaling the time since the last response.

*Behavioral measures:* Each session was divided into five 36-min parts ("segments") in order to monitor intra-session changes in the behavior. For each segment, each lever press was recorded as a function of time since last response (inter-response time, IRT). Further, the number of reinforcers delivered; and the number of correct (reinforcer present) and incorrect (no reinforcer present) lid openings were recorded for each segment.

The total number of lever presses is an expression of the general activity level and therefore a measure of degree of *overactivity.* The percentage of correct lever choices of the total number of lever presses when the reinforcers are delivered infrequently is a measure of *sustained attention.* The number of responses with short IRTs (<0.33 s) is a measure of degree of *impulsiveness* ("cannot hold back a response even when one knows it is an unnecessary one").

*Drug administration:* Administration of the drugs started following behavioral stabilization at session 49. The two isomers of amphetamine and combinations were compared with vehicle and with each other. All rats received d-amphetamine sulphate and 1-amphetamine sulphate. The dosing was balanced in the initial schedule. The schedule was extended to take into account results obtained by session 76. This involved repeating some of the previous results when the previous ones had deviated from what was predicted and adding new doses that the obtained data suggested might be of interest: 0.5 mg/kg d-amphetamine, 1 mg/kg 1-amphetamine, 0.75 mg/kg d-amphetamine + 0.5 mg/kg 1-amphetamine, 0.25 mg/kg d-amphetamine +1.5 mg/kg l-amphetamine. Each rat was dosed intraperitoneally -30 min before testing, with either vehicle (saline) or drug. Drugs were administered every 3^{rd} or 4^{th} day.

*Drugs:* The drugs were from Boeringer-Ingelheim US. *Doses* were 0.5, 1.0, and 1.5 mg/kg for d-amphetamine; and 1.0, 2.0, and 3.0 mg/kg for 1-amphetamine. The combinations were 0.75 mg/kg D (d-amphetamine) + 0.5 mg/kg L (1-amphetamine), 0.5 mg/kg D + 1 mg/kg L, 0.25 mg/kg D + 1.5 mg/kg L, and 0.5 mg/kg D + 2 mg/kg L.

*Data management and statistical procedures:* The mean behavior is regarded as the drug response, and dose-response curves are plotted for each drug and strain. Preliminary analyses of the data are processed by univariate analyses of variance (ANOVAs) with the Statistica 5.5 program (StatSoft, 1993). Within-subject variables are drug and dose. Strain is a between-subject variable. Univariate ANOVAs with Greenhouse-Geisser corrections of the univariate Fs are reported in order to correct for repeated measures within subjects.

### Results

*General:* The results show pronounced impulsiveness, overactivity and less efficient sustained attention in the SHR model of ADHD. The drugs gave clear dose-response curves. There was no indication of stereotypic or severely drugged behavior following any dose in any of the strains. For the case of SHR, d-amphetamine was about twice as potent as 1-amphetamine. The dose-response-curves were different for the different behaviors. D-amphetamine affected general activity level and impulsiveness more than 1-amphetamine. Low doses of 1-amphetamine, however, improved sustained attention more markedly than low doses of d-amphetamine. As seen in increased variability during non-injection sessions, sustained attention was adversely affected in the control group. These results are supported by the drug combinations tested.

*Acquisition:* As is the case in children with ADHD, the symptoms develop by time, but differently for the different behaviors. The final schedule was installed on session 13. A pronounced overactivity was seen in SHR from this session on. SHR impulsiveness, responding within 0.67 sec since the previous lever press (although such a lever press was almost never reinforced), continued to increase in the SHR throughout the entire study. This measure was accompanied by increased variability something that is typical in ADHD. In order to obtain more equal variances required by the ANOVAs, impulsiveness was therefore subjected to a log10-transformation. Sustained attention improved in the WKY until about session 50. In the SHR, however, sustained attention started to improve again following a few sessions with drug. These drug effects may, however, have been due to the late improvement in performance. The data were therefore transformed. The most suitable transformation found for these data was: *Transformed Percent Correct =Real Percent Correct-(Session Number *0.15).* This transformation apparently did not alter the relative behavioral differences between the two groups. These transformations were used for evaluating drug effects. Drug experience apparently had negative effects on WKY behavior (in terms of more variable performance) during sessions without active drug.

### Effects of drugs

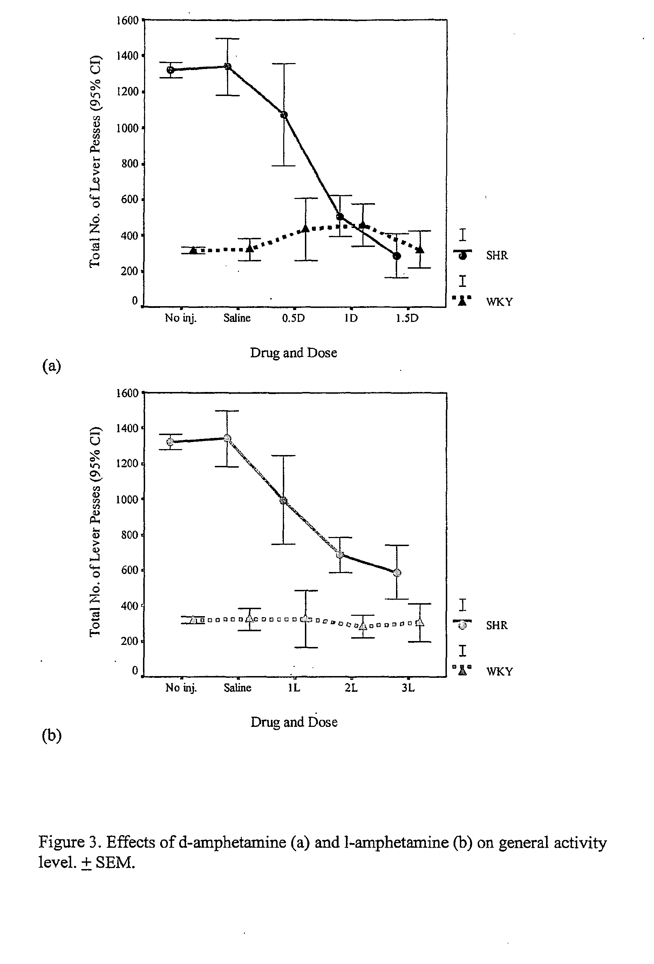

*Overactivity.* The pronounced SHR overactivity was reduced by both drugs (Figure 3). Following the highest doses of either drug, the general activity level of the SHR approached that of the WKY, more so after the highest dose of d-amphetamine than after 1-amphetamine. WKY behavior was unaffected by the drugs.

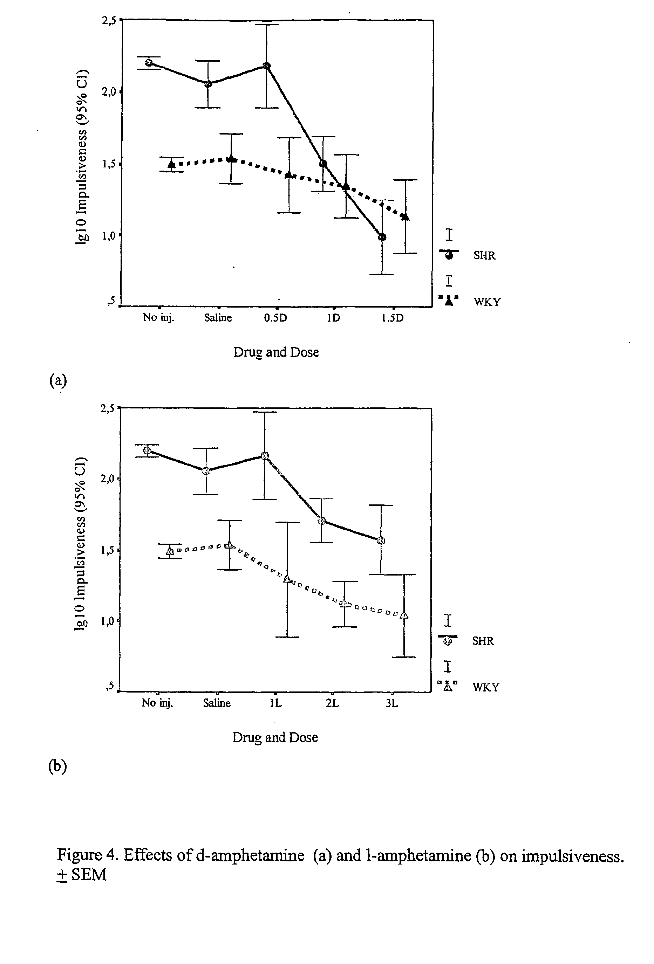

*Impulsiveness.* The SHR showed a pronounced impulsiveness that was reduced by both the drugs although 1-amphetamine was less efficient than d-amphetamine (Figure 4). WKY behavior was unaffected by the drugs.

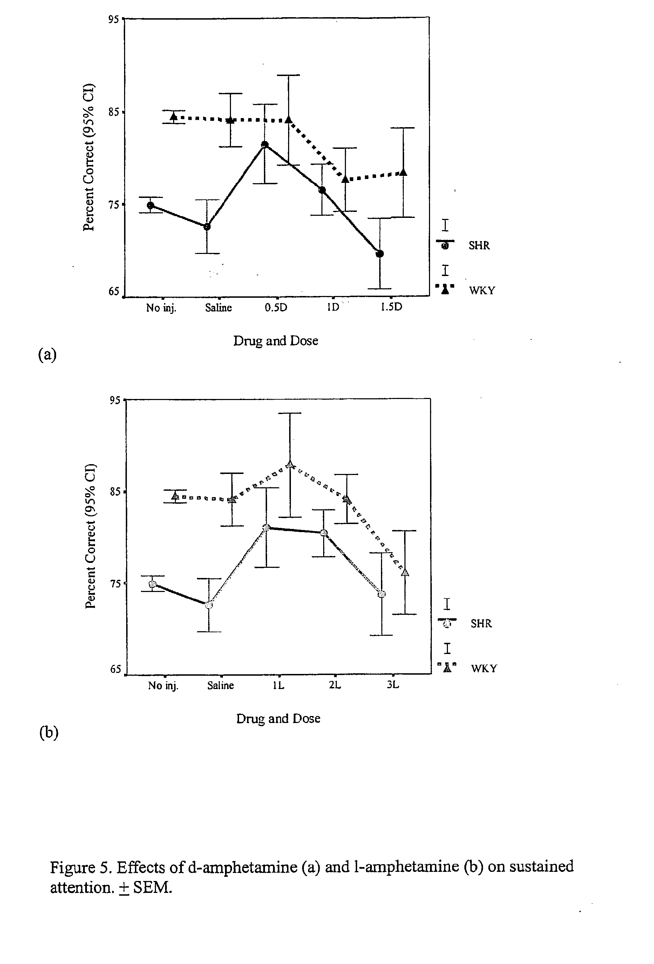

*Sustained attention.* Without medication, the SHR showed poorer sustained attention than WKY controls. In contrast to the effects on activity and impulsiveness where the normalization was seen following the highest doses used, sustained attention appeared to improve in the SHR following low doses (Figure 5).

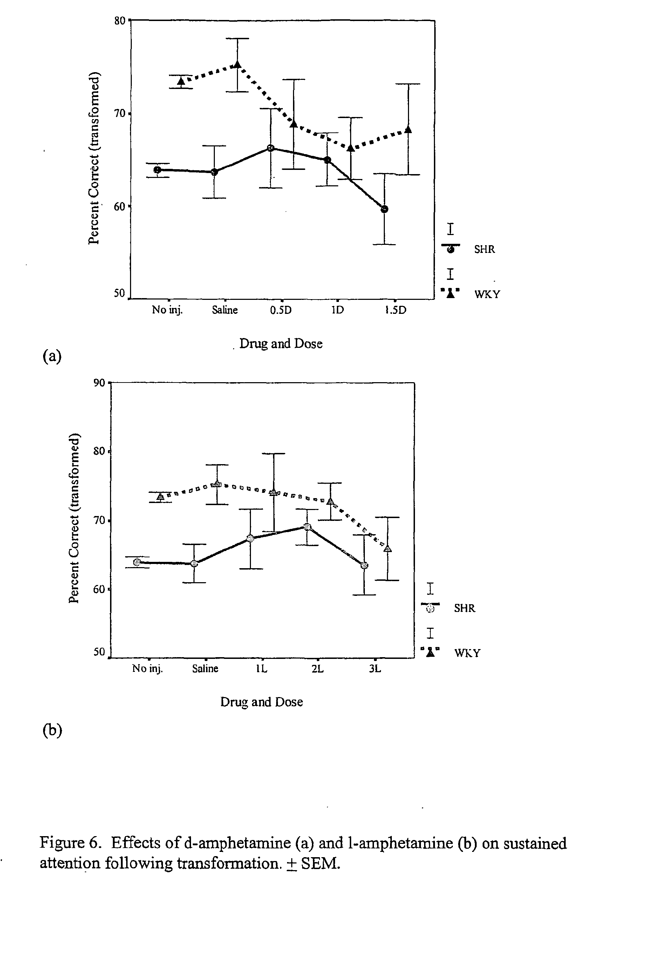

The improved sustained attention in the SHR behavior may be due to the mentioned changing baseline following some time with drug. Therefore, new analyses were performed on transformed data in order to take the gradually changing baseline into account. Improved performance is still present in the SHR group following the lowest doses of 1-amphetamine (Figure 6). Following this correction, the SHR improvement following d-amphetamine disappeared. 1-Amphetamine, with saline as baseline, still produced a significant improvement in the SHR group.

*Reinforcers delivered.* The mult RI180 EXT schedule used in the present study was programmed so that even large group differences in lever pressing will result in approximately 30 reinforcers (drops of water) during a 90-min session, also for the case of the less active group. A major advantage of such a schedule is the fact that systematic group differences in quenching thirst should not be of concern when interpreting the other data. The results show that both groups received ~30 reinforcers also following the highest doses.

*Stereotypy*/*severely drugged behavior:* No animal appeared drugged at any time in this study. Despite the fact that only 5 sec was available for collecting the reinforcer, the animals in both groups collected all the reinforcers delivered except an occasional one following the highest doses in the WKY and following the 1.5 mg/kg d-amphetamine dose in the SHR. Further, there was never any nose bleeding in any of the animals.

### Effects of combining the drugs

In order to test whether combinations of the amphetamine isomers produce additive or synergistic effects, the following combinations were tested and compared with equivalent doses of the single enantiomers, assuming that d-amphetamine is twice as potent as 1-amphetamine (which was shown in a previous experiment): (A) 0.75 mg/kg D (d-amphetamine) + 0.5 mg/kg L (1-amphetamine), 0.5 mg/kg D + 1 mg/kg L, 0.25 mg/kg D + 1.5 mg/kg L. These combinations were compared to 1 mg/kg D and 2 mg/kg L. (B) 0.5 mg/kg D + 2 mg/kg L, compared to 1.5 mg/kg D and 3 mg/kg L. It is predicted that the doses within (A)-combinations should be equipotent and the ones within (B)-combinations should be equipotent.

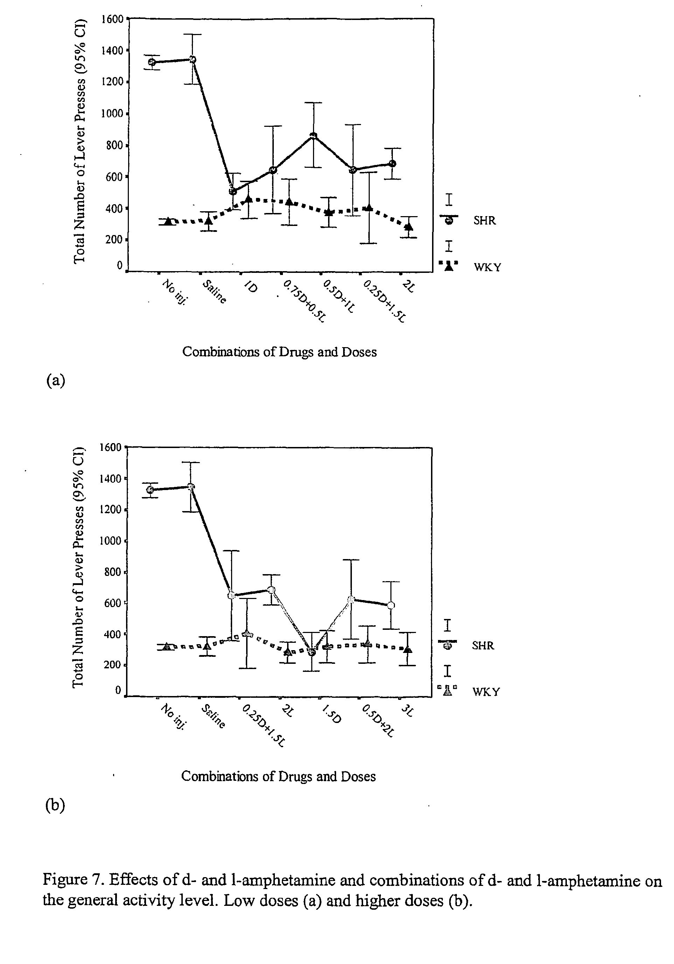

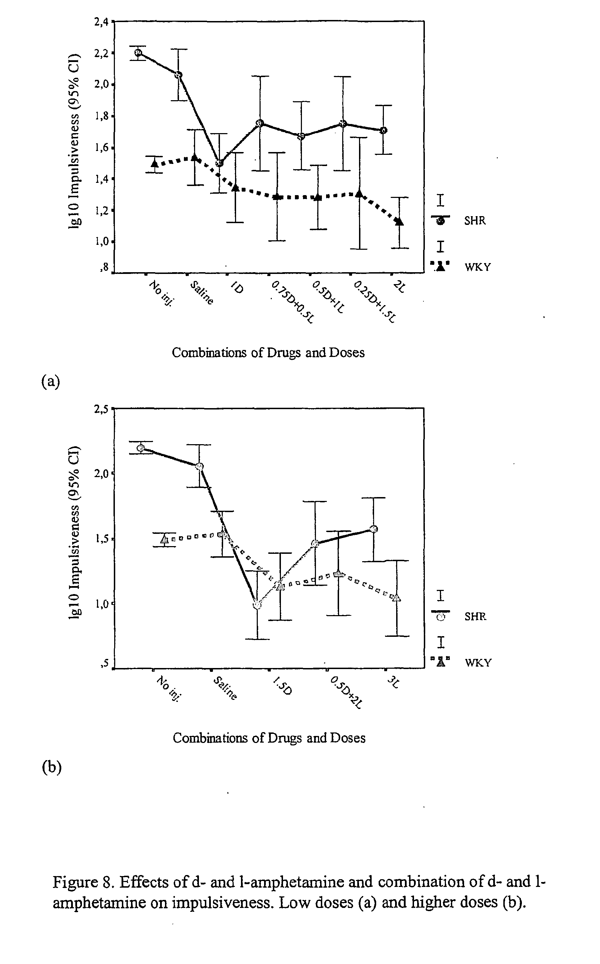

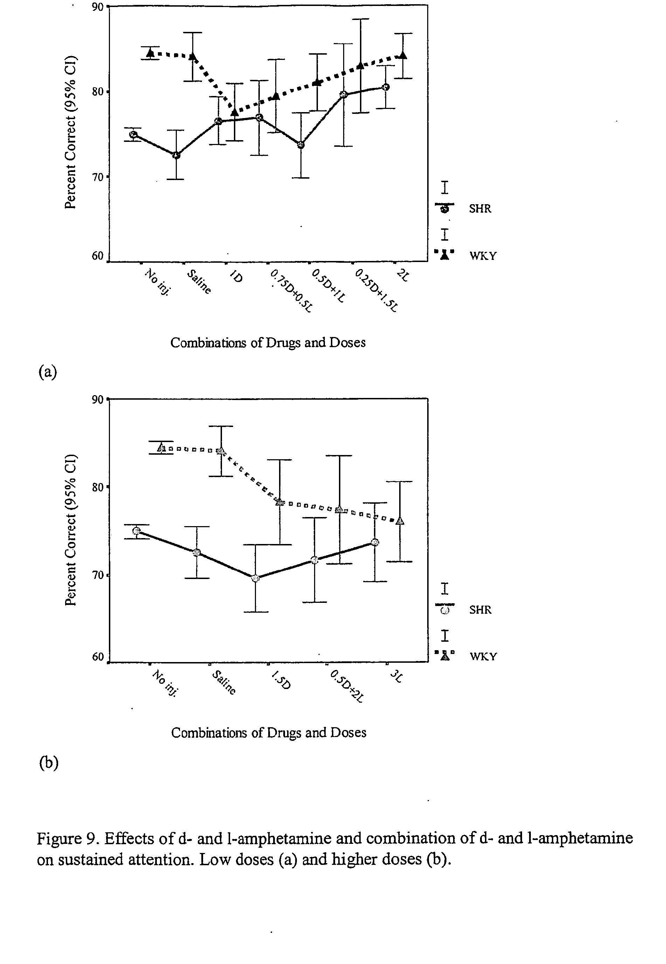

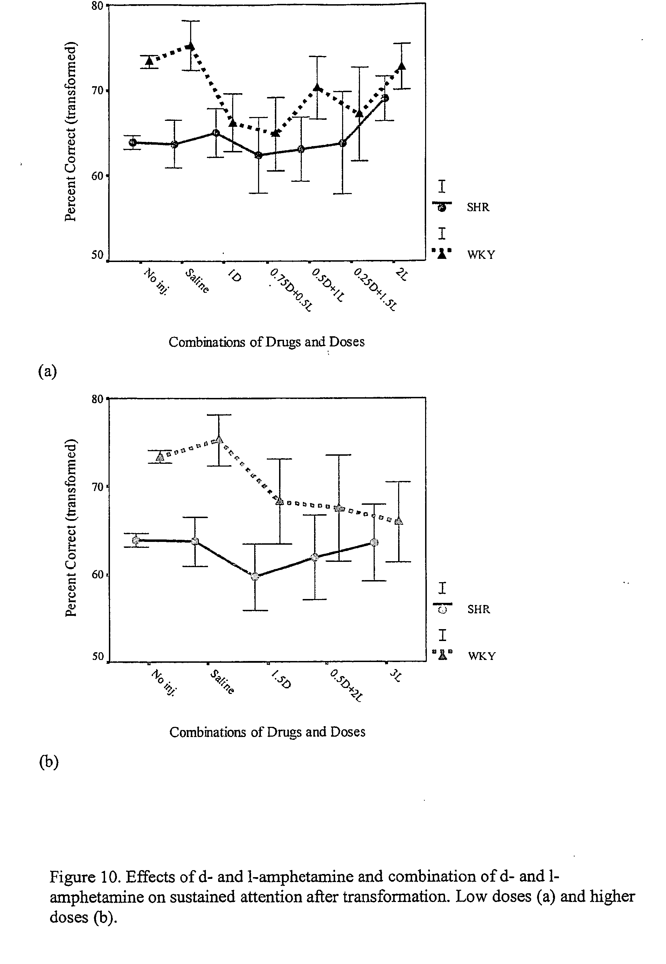

Although the effects are small, the results show that a higher fraction of d-amphetamine was more efficient for reducing overactivity and impulsiveness in the SHR (Figures 7 and 8). In contrast, a higher fraction of 1-amphetamine is more efficient in improving sustained attention (Figures 9 and 10). The drugs, particularly d-amphetamine, produced slight response stimulation in the WKY and, more significantly, *deteriorated* sustained attention in the WKY controls.

*Overactivity.* The result showed that a higher fraction of d-amphetamine more efficiently reduced SHR overactivity (Figure 7).

*Impulsiveness.* The result showed that, in particular for the highest dose combination, a higher fraction of d-amphetamine more efficiently reduced SHR impulsiveness (Figure 8).

*Sustained attention.* There was no significant 2-way interaction between strain x combination, ps > .20, for any dose on the untransformed measures. These results show additive effects of the two drugs (Figure 9).

The statistical results for the transformed measure of sustained attention were quite similar to the ones for the untransformed. There was no significant 2-way interaction between strain x combination, ps > .20, showing additive effects of the two drugs added (Figure 10). However, this result does not refute the result that SHR performed better following 2 mg/kg 1-amphetamine than following saline, t(15)=3,03996, p <0.01), T-test with dependent samples.

### Discussion

The amphetamines affected all behaviors tested. The results showed clearer dose-response curves in the SHR than in the WKY. The conclusion from previous studies that d-amphetamine is about twice as potent as 1-amphetamine in reducing hyperactivity and impulsivity, is to a large extent supported. Low doses of l-amphetamine improved sustained attention while higher doses of both isomers reduced overactivity and impulsiveness. A higher fraction of d-amphetamine was more efficient for reducing overactivity and impulsiveness in the SHR. In contrast, a higher fraction of l-amphetamine is more efficient in improving sustained attention.

### REFERENCE LIST FOR EXAMPLE 3

Catania, A. C. (1998) Learning (4th edition. ed.) Prentice Hall,Upper Saddle River. http://vig.prenhall.com/catalog/academic/product/1,4096,0132352508,00.html

Sagvolden, T. (2000) Behavioral validation of the spontaneously hypertensive rat (SHR) as an animal model of attention-deficit/hyperactivity disorder (AD/HD). Neuroscience and Biobehavioral Reviews 24: 31-39. PM:4:0010654658

Sagvolden, T. (2001) The spontaneously hypertensive rat as a model of ADHD. In: Stimulant drugs and ADHD: Basic and clinical neuroscience: pp. 221-237, M.V.Solanto, A. F. T. Amsten, & F. X. Castellanos (Eds.), Oxford University Press. http://www.oup.co.uk/isbn/0-19-513371-4

Sagvolden, T., Aase, H., Johansen, E. B., & Farshbaf, M. (2004a) Animal Models of Attention-Deficit/Hyperactivity Disorder. In: Cognitive and Affective Neuroscience of Psychopathology D.Barch (Ed.), Oxford University Press.

Sagvolden, T., Johansen, E. B., Aase, H., & Russell, V. A. (2004b). A Dynamic Developmental Theory of Attention-Deficit/Hyperactivity Disorder (ADHD) Predominantly Hyperactive/Impulsive and Combined Subtypes. Behavioral and Brain Sciences Submitted.

StatSoft, I. (1993). STATISTICA for Windows. Tulsa: OK: StatSoft Inc.

### Example 4: Effect of amphetamines on % distribution of wake versus sleep time

The effects of 4 different forms of amphetamine (d- and 1-amphetamine sulphate and the two racemic forms dl-amphetamine sulphate and dl-amphetamine aspartate) on the sleep-wake cycle were evaluated in conscious freely-moving male rats, which were Rj: Wistar (Han) rats, weighing 333 - 375 g at the beginning of the implantations. They were supplied by Elevage Janvier, 53940 Le Genest-Saint-Isle, France.

Implanted animals were individually placed in transparent perspex boxes in a soundproof, light-controlled, ventilated cabin and were habituated to the recording environment for at least 10 days. Drug-free baseline EEG was recorded for 23 hours. Then the rats were administered vehicle and the EEG recorded for another 23 hours. The next day, rats were treated with one of the 4 amphetamine forms first at the dose of 3 mg/kg, than at the dose of 1 mg/kg. The following variables were measured: latency to the appearance of stable slow-wave sleep (SWS), latency to the first appearance of rapid-eye-movement sleep (REM), the number of REM sleep phases, and the distribution percentage of wake/SWS/REM (sleep architecture).

Test sessions following treatment were individually compared with corresponding control sessions. As no differences were apparent after vehicle, the 2 control sessions with and without vehicle, were averaged.

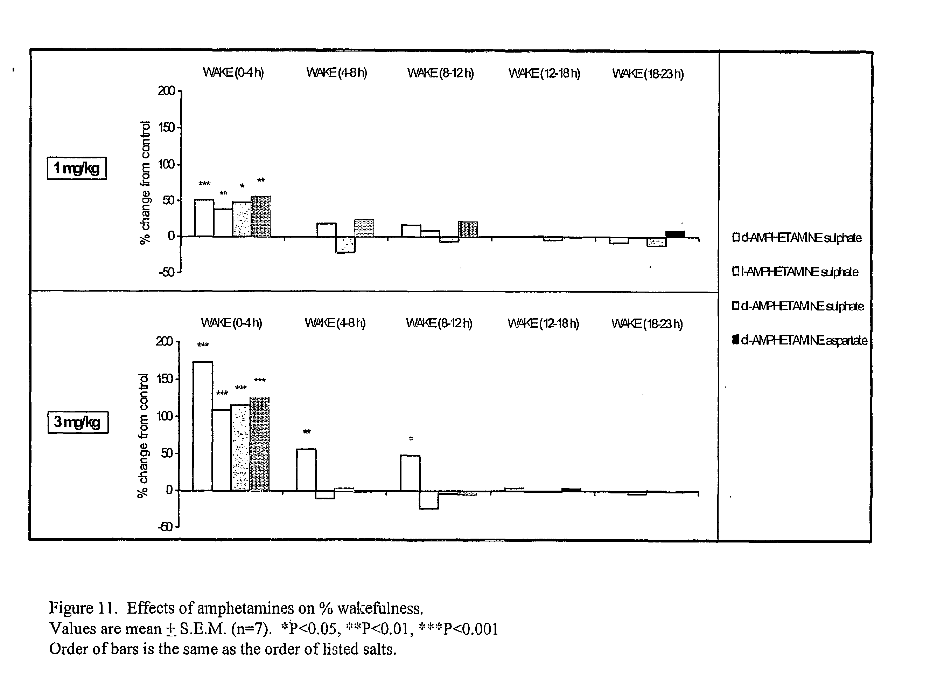

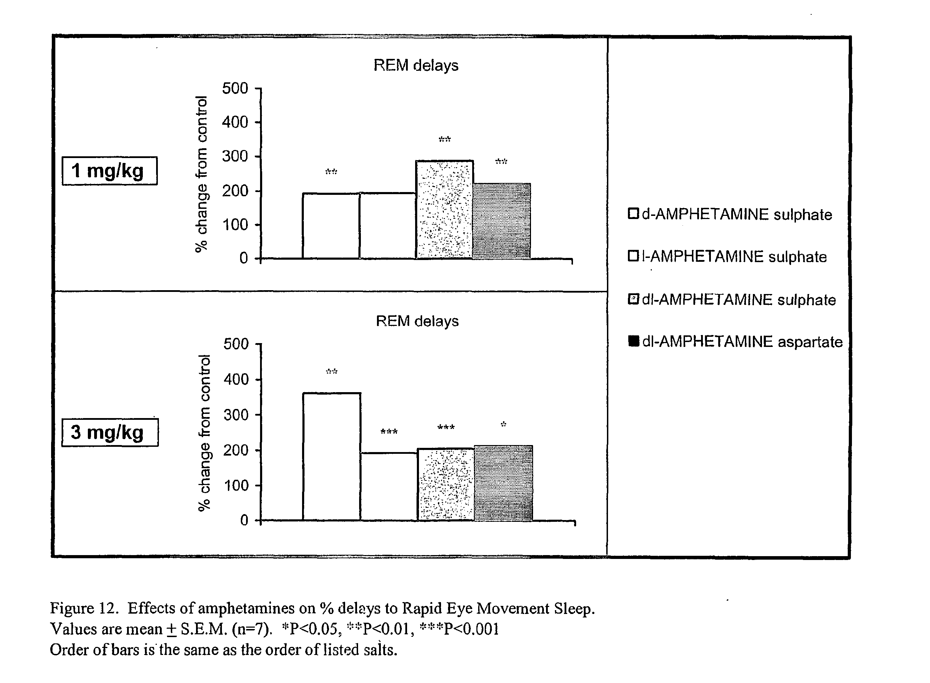

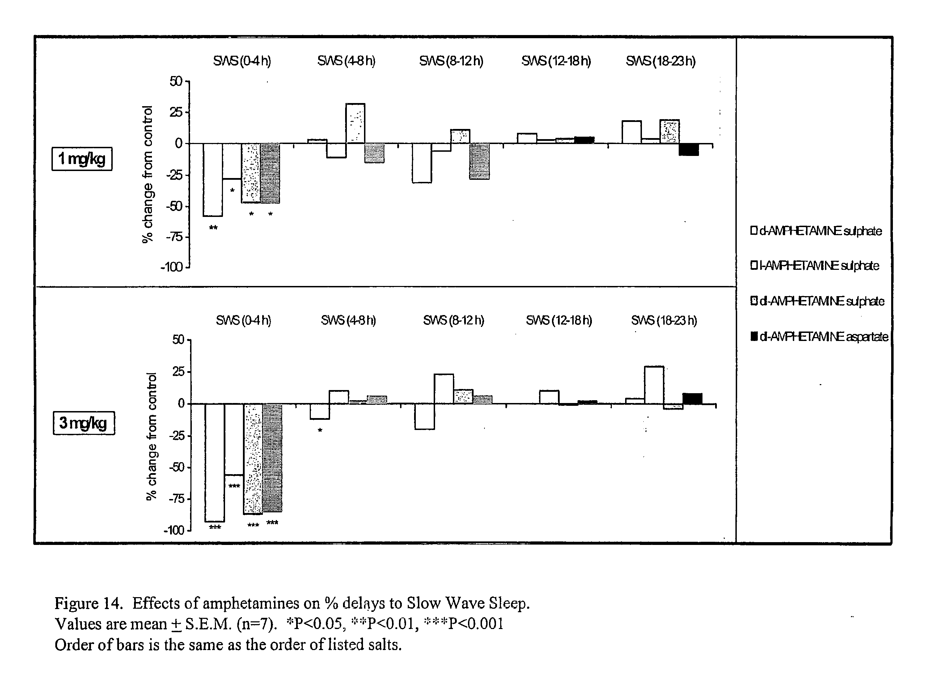

All 4 amphetamine delayed onset to SWS (Figure 14) and REM (Figure 12) sleep at both doses tested. The delays to onset of SWS were longer at 3 mg/kg than at 1 mg/kg. The delays to onset of REM sleep were similar for both doses, except for d-amphetamine sulphate at 3 mg/kg for which the delay tended to be longer. All amphetamine forms increased waking (Figure 11) and reduced SWS and REM sleep over the first 4 h of recording. The effects were more marked at 3 mg/kg than 1 mg/kg and the effects of d-amphetamine sulphate at 3 mg/kg were significantly greater than the 1-isomer on SWS and wake. All amphetamine forms reduced REM sleep between 4 and 8 h after administration at 3 mg/kg and dl-amphetamine aspartate also at 1 mg/kg. d-Amphetamine sulphate at 3 mg/kg increased waking and reduced SWS at this time point. When measured over the first 12 h of recording, all amphetamine forms reduced SWS and REM sleep and increased waking at 3 mg/kg. The effects were similar at 1 mg/kg but less pronounced.

Overall, d-amphetamine was more potent than 1-amphetamine, and its effects were longer lasting.

### Example 5: Effect of amphetamines on weight gain and food intake in rats

Rats were given access to food and water for 3 hours per day. The acclimation period was 10 days. Thereafter, vehicle or doses of l- or d-amphetamine sulphate were administered to the rats once a day one hour before access to food and water. Body weight was measured every day (Figure 11) before the drug administration and then food intake (Figure 12) and water intake were measured over the 3 hours each day when the rats had access to the same.

There was little effect on water intake. As can be seen in the figures, d-amphetamine was more potent than l-amphetamine. Both the d- and l-amphetamines produced dose-related reductions in body weight gain. D-amphetamine decreased food intake over the measured 10 day period, while the 1-amphetamine decreased food intake over the first 1-2 days only.

### Example 6 - Pharmaceutical combinations

A) Multi-laminated beads - Delayed-release beads containing amphetamines having l/d-isomer ratios of 1/1, 2/1, 3/1, 4/1 or containing only 1-isomer are overcoated with racemic amphetamine or amphetamines that have a d/l ratio of 3/1, e.g., as in Adderall^{®}, or with d-amphetamine alone to form a one-bead system with a total daily dose of 10, 20, 30, 40, 50 or 60 mg.
B) Combination of delayed-release beads containing amphetamines having l/d-isomer ratios of 1/1, 2/1, 3/1, 4/1 or containing only l-isomer and instant-release powder blend containing racemic amphetamine or amphetamines that have a d/l ratio of 3/1, e.g., as in Adderall^{®}, or d-amphetamine alone, with a total daily dose of 10, 20, 30, 40, 50 or 60 mg.

The delayed-release beads containing l-enhanced amphetamines are either encapsulated with the instant-release powder blend into hard gelatin capsules or compressed with the instant-release powder blend into tablets. Bi-layer tablets, wherein one layer is for the instant-release amphetamines and the other layer is for the l-enhanced amphetamine delayed-release beads formulated with processing aids, such as cushion materials, are also formed.

### C) Delayed-release tablets

Amphetamines having l/d-isomer ratios of 1/1, 2/1, 3/1, 4/1 or only 1-isomer are compressed with pharmaceutical excipients, such as lubricants, disintegrants, glidants, bulking agents, and binding agents. Subsequently, the tablets can be coated with a polymeric layer, which provides the lag time for a pulsatile delivery. The delayed-release tablet(s) can also be encapsulated with instant-release powder blend containing racemic amphetamine or amphetamines that have a d/l ratio of 3/1, e.g., as in Adderall^{®}, or d-amphetamine alone with a total daily dose of 10, 20, 30, 40, 50 or 60 mg, into hard gelatin capsules. The amphetamines in the instant release powder blend can also be coated over the delayed-release tablets. Additionally, it is possible to use an inlaid tablet design to incorporate the delayed-release tablet into an instant-release matrix.

### D) Compression coating

A barrier layer can be compressed over a tablet that contains amphetamines having l/d-isomer ratios of 1/1, 2/1, 3/1, 4/1 or only 1-isomer using a compression coating press. The compression coated tablets can be overcoated with racemic amphetamine or amphetamines that have a d/l ratio of 3/1, e.g., as in Adderall^{®}, or d-amphetamine alone with a total daily dose of 10, 20, 30, 40, 50 or 60 mg, or can be encapsulated into hard gelatin capsules with an instant-release powder blend of such d-enhanced amphetamines.

### E) Osmotic tablets with a blank layer

A blank layer in osmotic tablets can provide a lag time for pulsatile drug delivery. Two layered osmotic tablets, which comprise one blank layer and one layer of amphetamines having l/d-isomer ratios of 1/1, 2/1, 3/1, 4/1 or only l-isomer, are overcoated with racemic amphetamine or amphetamines that have a d/l ratio of 3/1, e.g., as in Adderall^{®}, or d-amphetamine alone with a total daily dose of 10, 20, 30, 40, 50 or 60 mg, to form a complete system. Three layered osmotic tablets can also be formed wherein a blank layer is inserted between the two types of amphetamines layers to achieve the pulsatile drug delivery.
F) A sugar core is coated with l-enhanced amphetamines as described above in this example then coated with polymer and amphetamines which are 5%, 10%, or 20% lower than the dose on the next outer layer, and have an l/d isomer ratio that is the same as in the next outer layer or the same as in the first layer on the core, or is between the two, then coated with polymer and amphetamines that are d-enhanced as described and then optionally coated with polymer only. The sugar core and the first layer of l-enhanced amphetamines can be replaced with a bead or tablet that contains the amphetamine and the remaining coatings are then applied thereon as described above.
G) A tablet or capsule is formed that contains multiple types of beads as described above having differing timings of release of amphetamines and/or different rates of release of amphetamines of either d-enhanced or l-enhanced configurations.

### Example 7

| Ingredients: | |
|---|---|
| l-amphetamine sulfate | 200 gram |
| microcrystalline cellulose | 500 gram |
| lactitol | 280 gram |
| silicon dioxide | 10 gram |
| magnesium stearate | 10 gram |

The amphetamine salts, lactitol, microcrystalline cellulose, and silicon dioxide are sieved through a 60 mesh screen and loaded into a V-shaped blender with an intensifier bar. The powder mixture is blended for 15 min. with the intensifier bar on for 3 min. at the middle of the blending process. The powder blend is unloaded and screened through a 60 mesh sieve. The screened powder blend is lubricated with magnesium stearate in the V-shaped blender for 3 min. The lubricated powder blend is compressed into tablets of different strengths using appropriate tablet tooling.

### Example 8

| Ingredients: | |
|---|---|
| l-amphetamine sulfate core tablets from Example 7 | 500 gram |
| Eudragit FS30D | 106.79 gram |
| triethyl citrate | 5.19 gram |
| talc | 13.03 gram |
| water | 147.03 gram |

The coating dispersion is prepared by dispersing triethyl citrate, talc, and Eudragit FS30D into water and mixing for at least 30 min. Under suitable fluidization conditions, the coating dispersion is sprayed onto the core tablets in a side-vented pan coater.

### Example 9

| Ingredients: | |
|---|---|
| l-amphetamine sulfate | 200 gram |
| microcrystalline cellulose | 500 gram |
| lactitol | 280 gram |
| silicon dioxide | 10 gram |
| magnesium stearate | 10 gram |
| polyethylene glycol 8000 | 2970 gram |
| magnesium stearate | 30 gram |

The amphetamine salts, lactitol, microcrystalline cellulose, and silicon dioxide are sieved through a 60 mesh screen and loaded into a V-shaped blender with an intensifier bar. The powder mixture is blended for 15 min. with the intensifier bar on for 3 min. in the middle of the blending process. The powder blend is unloaded and screened through a 60 mesh sieve. The screened powder blend is lubricated with magnesium stearate in the V-shaped blender for 3 min. Polyethylene glycol 8000 is milled through a 40 mesh screen and the screened polyethylene glycol is lubricated with magnesium stearate in a V-shaped blender for 3 min. Using a compression coating press, 1-amphetamine sulfate powder blend is compressed into core tablets and polyethylene glycol 8000 is compressed around the core tablets to form a barrier layer.

### Example 10

| Ingredients: | |
|---|---|
| mixed amphetamine salts (d/l ratio 3: 1) | 200 gram |
| microcrystalline cellulose | 500 gram |
| lactitol | 280 gram |
| silicon dioxide | 10 gram |
| magnesium stearate | 10 gram |
| poly(ethylene oxide) | 680 gram |
| microcrystalline cellulose | 300 gram |
| silicon dioxide | 10 gram |
| magnesium stearate | 10 gram |
| 1-amphetamine sulfate | 200 gram |
| microcrystalline cellulose | 500 gram |
| lactitol | 280 gram |
| silicon dioxide | 10 gram |
| magnesium stearate | 10 gram |

The mixed amphetamine salts, lactitol, microcrystalline cellulose, and silicon dioxide are sieved through a 60 mesh screen and loaded into a V-shaped blender with an intensifier bar. The powder mixture is blended for 15 min. with the intensifier bar on for 3 min. at the middle of the blending process. The powder blend is unloaded and screened through a 60 mesh sieve. The screened powder blend is lubricated with magnesium stearate in the V-shaped blender for 3 min. Poly(ethylene oxide), microcrystalline cellulose, and silicon dioxide are screened through a 60 mesh screen and loaded into a V-shaped blender. The powder mixture is blended for 15 min. and lubricated with magnesium stearate for 3 min. L-amphetamine sulfate is also blended with other ingredients using a similar blending procedure. Three different powder blends are loaded onto a three-layer press and compressed into three-layer tablets.

### Example 11

| Ingredients: | |
|---|---|
| Three-layer core tablets from Example 10 | 500 gram |
| cellulose acetate | 23.68 gram |
| polyethylene glycol 400 | 2.63 gram |
| acetone | 420.96 gram |
| methyl alcohol | 105.24 gram |

Cellulose acetate and polyethylene glycol are dissolved in the mixture of acetone and methyl alcohol. The core tablets are coated by spraying the cellulose acetate solution in a side-vented pan coater. A hole is drilled into the coated tablets on the mixed amphetamine layer side.

### Example 12 - Pharmaceutical combination for blister pack embodiment

A pharmaceutical combination is prepared as a blister pack for use in one day:

Two sets are prepared. One having three compartments and the other having two compartments. Each compartment is labelled as 1^{st}, 2^{nd} and 3^{rd} dose as appropriate. For the two compartment packs, designs include first dose of 10, 20, 30, 40, 50 or 60 mg of Adderall^{®} immediate release tablets or immediate release tablets of only d-amphetamine and for each such dose, the second compartment contains in separate embodiments, doses of total amphetamines which are the same as the first dose, but have 1/d-isomer ratios of 1/1, 2/1, 3/1, 4/1 or contain only 1-isomer; or contains, in separate embodiments, doses of total amphetamines which are 5%, 10%, or 20% lower than the first dose, and have 1/d isomer ratios of 1/1, 2/1, 3/1, 4/1 or contain only 1-isomer. Other designs include 10, 20, 30, 40, 50 or 60 mg of Adderall XR^{®} tablets in the first pack and second doses having the 1-isomer contents as descried above. In three compartment packs, in addition to the foregoing, the third dose can also have an 1-isomer content as described above.

## Claims

1. -A pharmaceutical combination comprising an effective amount for a day of l- and d- amphetamine each in base and/or salt form, and each adapted for release such that the molar ratio of l-amphetamine to d-amphetamine released therefrom in a time period later in the day is higher than said ratio released therefrom in a time period earlier in the day;
wherein the time period later in the day is at least one hour following the time period earlier in the day.

2. A pharmaceutical combination of claim 1, wherein said earlier period is the time before 1:00 pm of a given day and said later period is the time thereafter.

3. A pharmaceutical combination of claim 1, wherein said amphetamine released in said earlier period comprises substantially only d-amphetamine, racemic amphetamine, or a mixture of d- and 1-amphetamine having more d- than 1-amphetamine.

4. A pharmaceutical combination of claim 1, wherein the molar ratio released of d- to 1- amphetamine in said earlier period is 4/1 to 2/1.

5. A pharmaceutical combination of claim 1, wherein the molar ratio released of d- to 1- amphetamine in said earlier period is less than 1/1.

6. -A pharmaceutical combination of claim 1, wherein substantially only d-amphetamine is released in said early period.

7. A pharmaceutical combination of claim 1, wherein said amphetamine released in said earlier period comprises a mixture of d- and l-amphetamine having more l- than d-amphetamine.

8. A pharmaceutical combination of claim 1, wherein said amphetamine released in said later period comprises substantially only l-amphetamine, racemic amphetamine, or a mixture of d- and l-amphetamine having more l- than d-amphetamine.

9. A pharmaceutical combination of claim 1, wherein said amphetamine released in said later period comprises a mixture of d- and l-amphetamine having more l- than d-amphetamine.

10. A pharmaceutical combination of claim 1, wherein the molar ratio released of l- to d-amphetamine in said later period is 2/1 to 6/1.

11. A pharmaceutical combination of claim 1, wherein substantially only l-amphetamine is released in said later period.

12. A pharmaceutical combination of claim 1, wherein the total amphetamine dose per day is 1 to 200 mg.

13. A pharmaceutical combination of claim 1, which comprises two separate oral dosage forms, one identified to be administered at a time to provide amphetamine release in said earlier period and the other identified to be administered at a time to provide amphetamine release in said later period:

14. A pharmaceutical combination of claim 1, which comprises a single oral dosage form which provides amphetamine release in both said earlier and later periods.

15. A pharmaceutical combination of claim 1, which comprises a dosage form providing immediate release of d-amphetamine in said earlier period.

16. Use of effective amounts of the l- and d-isomers of amphetamine in the manufacture of a medicament for the treatment of ADHD in a patient, wherein the l-and d- isomers of amphetamine are each independently in free base and/or salt form, and each adapted for release such that the molar ratio of
l-amphetamine to d-amphetamine released from the medicament in a time period later in the day is higher than said ratio released from the medicament in a time period earlier in the day; and the molar ratio of
the total amount of l-isomer to the total amount of d-isomer to be administered per day is greater than 1:3.
wherein the time period later in the day is at least one hour following the time period earlier in the day.

17. The use according to claim 16, wherein doses are to be administered individually at different times or are to be administered once in a single staged-release dosage form.

18. The use according to claim 16, wherein doses are to be administered in one or more dosage forms that are either immediate release or pulse release dosage forms and/or sustained or controlled release dosage forms.

19. The use according to claim 18, wherein the sustained or controlled release dosage form or dosage forms contain the l isomer.

20. The use according to claim 16, wherein two doses of amphetamine are to be administered to the patient in a day, the first dose having an l to d isomer ratio of 1:3 or contains only d isomer, and the later dose having an l to d isomer ratio of greater than 1:1 or contains l isomer only.

21. The use according to claim 20, wherein the second dose contains l isomer only.

22. A pharmaceutical combination according to claim 1, wherein doses are to be administered individually at different times or are to be administered once in a single staged-release dosage form.

23. A pharmaceutical combination according to claim 1, wherein doses are to be administered in one or more dosage forms that are either immediate release or pulse release dosage forms and/or sustained or controlled release dosage forms.

24. A pharmaceutical combination according to claim 23, wherein the sustained or controlled release dosage form or dosage forms contain the I isomer.

25. A pharmaceutical combination according to claim 1, wherein two doses of amphetamine are to be administered to the patient in a day, the first dose having an I to d isomer ratio of 1:3 or contains only d isomer, and the later dose having an I to d isomer ratio of greater than 1:1 or contains I isomer only.

26. A pharmaceutical combination according to claim 25, wherein the second dose contains I isomer only.

27. The use of a pharmaceutical combination of claim 1 for the manufacture of a medicament for the treatment of ADHD in a patient.

28. The use of a pharmaceutical combination of claim 1 for the manufacture of a medicament for the treatment of ADHD in a human, wherein the effectiveness of treatment of the inattentiveness in an ADHD human patient later in the day by the l-isomer is as good as treatment with a corresponding molar amount of d-amphetamine and is accompanied by a lesser side effect of sleep deterioration and/or decrease in food intake.

## Patentansprüche

1. Pharmazeutische Zusammenstellung enthaltend die wirksame Tagesmenge von 1- und d-Amphetamin, jedes in Form der Base und/oder des Salzes, und jedes derart für die Freisetzung angepasst, dass das daraus freigesetzte molare Verhältnis von 1-Amphetamin zu d-Amphetamin in einer Zeitspanne später am Tag höher ist, als das daraus freigesetzte Verhältnis in einer Zeitspanne früher am Tag;
worin die Zeitspanne später am Tag wenigstens eine Stunde der nach der Zeitspanne früher am Tag folgt.

2. Pharmazeutische Zusammenstellung nach Anspruch 1,
worin die frühere Zeitspanne die Zeit vor 13:00 Uhr des angegebenen Tages ist und die spätere Zeitspanne die darauf folgende Zeit ist.

3. Pharmazeutische Zusammenstellung nach Anspruch 1, worin das in der früheren Zeitspanne freigesetzte Amphetamin im Wesentlichen nur aus d-Amphetamin, racemischem Amphetamin oder einer Mischung aus d- und 1-Amphetamin, welche mehr d- als 1-Amphetamin hat, besteht.

4. Pharmazeutische Zusammenstellung nach Anspruch 1, worin das freigesetzte molare Verhältnis von 1- zu d-Amphetamin in der früheren Zeitspanne 4/1 bis 2/1 beträgt.

5. Pharmazeutische Zusammenstellung nach Anspruch 1, worin das freigesetzte molare Verhältnis von 1- zu d-Amphetamin in der früheren Zeitspanne weniger als 1/1 beträgt.

6. Pharmazeutische Zusammenstellung nach Anspruch 1, worin in der früheren Zeitspanne im Wesentlichen nur d-Amphetamin freigesetzt wird.

7. Pharmazeutische Zusammenstellung nach Anspruch 1, worin das in der früheren Zeitspanne freigesetzte Amphetamin eine Mischung aus d- und 1-Amphetamin enthält, welche mehr 1- als d-Amphetamin hat.

8. Pharmazeutische Zusammenstellung nach Anspruch 1, worin das in der späteren Zeitspanne freigesetzte Amphetamin im Wesentlichen nur aus 1-Amphetamin, racemischem Amphetamin oder einer Mischung aus d- und 1-Amphetamin besteht, welche mehr 1- als d-Amphetamin hat.

9. Pharmazeutische Zusammenstellung nach Anspruch 1, worin das in der späteren Zeitspanne freigesetzte Amphetamin eine Mischung aus d- und 1-Amphetamin enthält, welche mehr 1- als d-Amphetamin hat.

10. Pharmazeutische Zusammenstellung nach Anspruch 1, worin das freigesetzte molare Verhältnis von 1- zu d-Amphetamin in der späteren Zeitspanne 2/1 bis 6/1 beträgt.

11. Pharmazeutische Zusammenstellung nach Anspruch 1, worin in der späteren Zeitspanne im Wesentlichen nur 1-Amphetamin freigesetzt wird.

12. Pharmazeutische Zusammenstellung nach Anspruch 1, worin die Gesamttagesdosis an Amphetamin 1 bis 200 mg beträgt.

13. Pharmazeutische Zusammenstellung nach Anspruch 1, welche zwei getrennte orale Dosierungsformen enthält, eine **gekennzeichnet**, um zu einer Zeit verabreicht zu werden, um Amphetaminfreisetzung in der früheren Zeitspanne bereitzustellen und die andere **gekennzeichnet**, um zu einer Zeit verabreicht zu werden, um Amphetaminfreisetzung in der späteren Zeitspanne bereitzustellen.

14. Pharmazeutische Zusammenstellung nach Anspruch 1, welche eine orale Einzeldosierungsform enthält, welche Amphetaminfreisetzung, sowohl in der früheren, als auch in der späteren Zeitspanne, bereitstellt.

15. Pharmazeutische Zusammenstellung nach Anspruch 1, welche eine Dosierungsform enthält, welche die sofortige Freisetzung von d-Amphetamin in der früheren Zeitspanne bereitstellt.

16. Verwendung einer wirksamen Menge der 1- und d-Isomeren von Amphetamin zur Herstellung eines Arzneimittels für die Behandlung von ADAH bei einem Patienten, worin die 1- und d-Isomeren des Amphetamins unabhängig voneinander in Form der freien Base und/oder des Salzes vorliegen, und jedes derart für die Freisetzung angepasst, dass das aus dem Arzneimittel freigesetzte molare Verhältnis von 1-Amphetamin zu d-Amphetamin in einer Zeitspanne später am Tag höher ist, als das aus dem Arzneimittel freigesetzte Verhältnis in einer Zeitspanne früher am Tag; und das molare Verhältnis der Gesamtmenge von 1-Isomer zu der Gesamtmenge an d-Isomer, welches pro Tag zu verabreichen ist, größer als 1:3 ist,
worin die Zeitspanne später am Tag wenigstens eine Stunde der nach der Zeitspanne früher am Tag folgt.

17. Verwendung gemäß Anspruch 16, worin die Dosen individuell zu verschiedenen Zeiten zu verabreichen oder einmal in einer stufenweise freisetzenden Einzeldosisform zu verabreichen sind.

18. Verwendung gemäß Anspruch 16, worin die Dosen in einer oder mehreren Dosierungsformen zu verabreichen sind, die entweder sofort freisetzende oder gepulst freisetzende Dosierungsformen und/oder verzögert oder kontrolliert freisetzende Dosierungsformen sind.

19. Verwendung gemäß Anspruch 18, worin die verzögert oder kontrolliert freisetzende Dosierungsform oder Dosierungsformen das 1-Isomer enthalten.

20. Verwendung gemäß Anspruch 16, worin zwei Dosen von Amphetamin an einen Patienten am Tag zu verabreichen sind, die erste Dosis ein 1 zu d Isomerverhältnis von 1:3 hat oder nur d-Isomer enthält, und die spätere Dosis ein 1 zu d Isomerverhältnis größer als 1:1 hat oder nur 1-Isomer enthält.

21. Verwendung gemäß Anspruch 20, worin die zweite Dosis nur 1-Isomer enthält.

22. Pharmazeutische Zusammenstellung gemäß Anspruch 1, worin die Dosen individuell zu verschiedenen Zeiten zu verabreichen oder einmal in einer stufenweise freisetzenden Einzeldosisform zu verabreichen sind.

23. Pharmazeutische Zusammenstellung gemäß Anspruch 1, worin die Dosen in einer oder mehreren Dosierungsformen zu verabreichen sind, die entweder sofort freisetzende oder gepulst freisetzende Dosierungsformen und/oder verzögert oder kontrolliert freisetzende Dosierungsformen sind.

24. Pharmazeutische Zusammenstellung gemäß Anspruch 23, worin die verzögert oder kontrolliert freisetzende Dosierungsform oder Dosierungsformen das 1-Isomer enthalten.

25. Pharmazeutische Zusammenstellung gemäß Anspruch 1, worin zwei Dosen von Amphetamin an einen Patienten am Tag zu verabreichen sind, die erste Dosis ein 1 zu d Isomerverhältnis von 1:3 hat oder nur d-Isomer enthält, und die spätere Dosis ein 1 zu d Isomerverhältnis größer als 1:1 hat oder nur 1-Isomer enthält.

26. Pharmazeutische Zusammenstellung gemäß Anspruch 25 worin die zweite Dosis nur 1-Isomer enthält.

27. Verwendung einer pharmazeutische Zusammenstellung nach Anspruch 1, zur Herstellung eines Arzneimittels für die Behandlung von ADHD bei einem Patienten.

28. Verwendung einer pharmazeutische Zusammenstellung nach Anspruch 1, zur Herstellung eines Arzneimittels für die Behandlung von ADHD bei einem Menschen, worin die Wirksamkeit der Behandlung der Unaufmerksamkeit bei einem menschlichen ADHD-Patienten später am Tag durch das 1-Isomer so gut ist, wie eine Behandlung mit einer entsprechenden molaren Menge von d-Amphetamin und von weniger Nebenwirkungen durch Schlafstörungen und/oder der Verminderung der Nahrungsaufnahme begleitet ist.

## Revendications

1. Une combinaison pharmaceutique comprenant une quantité efficace journalière d'amphétamine l et d, chacune sous la forme d'une base et/ou d'un sel, et chacune adaptée pour une libération telle que le rapport molaire de l'amphétamine l à l'amphétamine d libéré pendant une période de temps plus tard dans la journée est supérieur au dit rapport libéré pendant une période dans le temps plus tôt dans la journée;
dans laquelle la période de temps plus tard dans la journée se situe au moins une heure après la période de temps plus tôt dans la journée.

2. Une combinaison pharmaceutique selon la revendication 1, dans laquelle ladite période plus tôt se situe avant 13 heures d'un jour donné et ladite période plus tard se situe après.

3. Une combinaison pharmaceutique selon la revendication 1, dans laquelle ladite amphétamine libérée pendant la période plus tôt comprend essentiellement seulement de l'amphétamine d, de l'amphétamine racémique, ou un mélange d'amphétamine d et l ayant plus d'amphétamine d que d'amphétamine l.

4. Une combinaison pharmaceutique selon la revendication 1, dans laquelle le rapport molaire de l'amphétamine d à l'amphétamine I libéré pendant ladite période plus tôt est de 4/1 à 2/1.

5. Une combinaison pharmaceutique selon la revendication 1, dans laquelle le rapport molaire de l'amphétamine d et l'amphétamine l libéré pendant la période plus tôt est inférieure à 1/1.

6. Une combinaison pharmaceutique selon la revendication 1, dans laquelle essentiellement seulement l'amphétamine d est libérée pendant ladite période plus tôt.

7. Une combinaison pharmaceutique selon la revendication 1, dans laquelle ladite amphétamine libérée pendant ladite période plus tôt comprend un mélange d'amphétamine d et l ayant plus d'amphétamine l que d'amphétamine d.

8. Une combinaison pharmaceutique selon la revendication 1, dans laquelle ladite amphétamine libérée pendant ladite période plus tard comprend essentiellement seulement de l'amphétamine l, de l'amphétamine racémique, ou un mélange d'amphétamine d et l ayant plus d'amphétamine l que d'amphétamine d.

9. Une combinaison pharmaceutique selon la revendication 1, dans laquelle ladite amphétamine libérée pendant ladite période plus tard comprend un mélange d'amphétamine d et l ayant plus d'amphétamine l que d'amphétamine d.

10. Une combinaison pharmaceutique selon la revendication 1, dans laquelle le rapport molaire de l'amphétamine l à l'amphétamine d libéré pendant ladite période plus tard est de 2/1 à 6/1.

11. Une combinaison pharmaceutique selon la revendication 1, dans laquelle essentiellement seulement l'amphétamine l est libérée pendant ladite période plus tard.

12. Une combinaison pharmaceutique selon la revendication 1, dans laquelle toute la dose journalière d'amphétamine est de 1 à 200 mg.

13. Une combinaison pharmaceutique selon la revendication 1, qui comprend deux formes de dosage distinctes pour voie orale, l'une identifiée pour être administrée à un moment pour obtenir la libération d'amphétamine pendant ladite période plus tôt et l'autre identifiée pour être administrée à un moment pour obtenir la libération d'amphétamine pendant ladite période plus tard.

14. Une combinaison pharmaceutique selon la revendication 1, qui comprend une seule forme de dosage pour voie orale qui donne une libération d'amphétamine aussi bien pendant ladite période plus tôt que pendant ladite période plus tard.

15. Une combinaison pharmaceutique selon la revendication 1, qui comprend une forme de dosage donnant une libération immédiate d'amphétamine d pendant ladite période plus tôt.

16. Utilisation de quantités efficaces d'isomères I et d d'amphétamine dans la fabrication d'un médicament pour le traitement de l'ADHD chez un patient, dans laquelle les isomères I et d d'amphétamine sont chacun indépendamment sous la forme d'une base libre et/ou d'un sel, et chacun adapté pour une libération telle que le rapport molaire de
l'amphétamine 1 à l'amphétamine d libéré par le médicament pendant une période de temps plus tard dans la journée est supérieur au dit rapport libéré par le médicament pendant une période de temps plus tôt dans la journée ; et le rapport molaire de
la quantité totale d'isomère I à la quantité totale d'isomère d à administrer par jour est supérieur à 1:3 ;
dans laquelle la période de temps plus tard dans la journée est au moins une heure après la période de temps plus tôt dans la journée.

17. L'utilisation selon la revendication 16, dans laquelle des doses doivent être administrées individuellement à différentes heures ou en une fois dans une seule forme de dosage à libération progressive.

18. L'utilisation selon la revendication 16, dans laquelle des doses doivent être administrées dans une ou des formes de dosage qui sont des formes de dosage soit à libération immédiate soit à libération fractionnée et/ou des formes de dosage à libération commandée ou soutenue.

19. L'utilisation selon la revendication 18, dans laquelle la forme de dosage ou les formes de dosage à libération commandée ou soutenue contiennent de l'isomère l.

20. L'utilisation selon la revendication 16, dans laquelle deux doses d'amphétamine doivent être administrées par jour au patient, la première dose ayant un rapport d'isomères I à d de 1:3 ou contenant seulement de l'isomère d, et l'autre dose ayant un rapport d'isomères I à d supérieur à 1:1 ou contenant seulement de l'isomère I.

21. L'utilisation selon la revendication 20, dans laquelle la deuxième dose contient seulement de l'isomère I.

22. Une combinaison pharmaceutique selon revendication 1, dans laquelle des doses doivent être administrées individuellement à différentes heures ou en une fois dans une seule forme de dosage à libération progressive.

23. Une combinaison pharmaceutique selon la revendication 1, dans laquelle des doses doivent être administrées dans une ou des formes de dosage qui sont des formes de dosage soit à libération immédiate soit à libération fractionnée et/ou des formes de dosage à libération commandée ou soutenue.

24. Une combinaison pharmaceutique selon la revendication 23, dans laquelle la forme de dosage ou les formes de dosage à libération commandée ou soutenue contiennent le l'isomère 1.

25. Une combinaison pharmaceutique selon la revendication 1, dans laquelle deux doses d'amphétamine doivent être administrées par jour au patient, la première dose ayant un rapport d'isomères I à d de 1:3 ou contenant seulement de l'isomère d, et l'autre dose ayant un rapport d'isomères 1 à d supérieur à 1:1 ou contenant seulement de l'isomère I.

26. Une combinaison pharmaceutique selon la revendication 25, dans laquelle la deuxième dose contient seulement de l'isomère I.

27. L'utilisation d'une combinaison pharmaceutique selon la revendication 1 pour la fabrication d'un médicament pour le traitement de l'ADHD chez un patient.

28. L'utilisation d'une combinaison pharmaceutique selon la revendication 1 pour la fabrication d'un médicament pour le traitement de l'ADHD chez un humain, dans laquelle l'efficacité du traitement, par l'isomère 1, du manque d'attention chez un patient humain souffrant d'ADHD plus tard dans la journée est aussi bonne que celle du traitement avec une quantité molaire d'amphétamine d correspondante et est accompagnée d'une diminution d'effet secondaire relatif à une détérioration du sommeil et/ou à une diminution de l'appétit.
